(19) Europäisches Patentamt
European Patent Office
Office européen des brevets

(11) EP 1 799 265 B1

# (12) EUROPÄISCHE PATENTSCHRIFT

(45) Veröffentlichungstag und Bekanntmachung des Hinweises auf die Patenterteilung:
**08.09.2010 Patentblatt 2010/36**

(21) Anmeldenummer: **05786251.8**

(22) Anmeldetag: **20.09.2005**

(51) Int Cl.:
***A61K 47/10*** *(2006.01)* ***A61K 47/12*** *(2006.01)*
***A61K 31/495*** *(2006.01)* ***A61K 31/18*** *(2006.01)*

(86) Internationale Anmeldenummer:
**PCT/EP2005/010143**

(87) Internationale Veröffentlichungsnummer:
**WO 2006/032461 (30.03.2006 Gazette 2006/13)**

## (54) STABILE DOSIERUNGSFORM VON PHENYLALANIN-DERIVATEN

STABLE DOSING FORM OF PHENYLALANINE DERIVATIVES

FORMES GALÉNIQUES STABLES DE DERIVÉS DE PHÉNYLALANINE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **21.09.2004 DE 102004045720**

(43) Veröffentlichungstag der Anmeldung:
**27.06.2007 Patentblatt 2007/26**

(73) Patentinhaber: **Wilex AG**
**81675 München (DE)**

(72) Erfinder:
- **SCHMALIX, Wolfgang**
  **82194 Gröbenzell (DE)**
- **BÜRGLE, Markus**
  **81369 München (DE)**
- **KOCH, Klaus**
  **86415 Mering (DE)**

(74) Vertreter: **Weiss, Wolfgang**
**Weickmann & Weickmann**
**Patentanwälte**
**Richard-Strauss-Strasse 80**
**81679 München (DE)**

(56) Entgegenhaltungen:
**WO-A-00/04954 WO-A-99/05096**
**WO-A-03/076391 WO-A-03/103644**

EP 1 799 265 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft verbesserte und stabile pharmazeutische Formulierungen von Phenylalanin-Derivaten und deren Verwendung als Urokinase-Inhibitoren, insbesondere zur Behandlung von malignen Tumoren und von Tumormetastasen.

Die Fähigkeit solider Tumoren zur Ausbreitung und Metastasierung in umgebendes Gewebe korreliert mit dem Abbau bzw. Umbau der extrazellulären Matrix (Tumorstroma) in der Umgebung der Tumorzelle bzw. mit deren Fähigkeit zur Durchdringung der Basalmembran. Obwohl die (patho-)biochemischen Zusammenhänge noch nicht endgültig aufgeklärt sind, kommt dem Plasminogenaktivator Urokinase (uPA) und dem Urokinaserezeptor (uPAR) eine zentrale Bedeutung zu. uPA vermittelt die proteolytische Spaltung von Plasminogen zu Plasmin. Plasmin wiederum ist eine Protease mit breitem Wirkspektrum, die Komponenten der extrazellulären Matrix wie Fibrin, Fibronektin, Laminin und das Proteingerüst der Proteoglykane direkt abzubauen vermag. Außerdem kann Plasmin "latente" Metalloproteasen und das inaktive Proenzym von uPA, pro-uPA, aktivieren.

Tumorzellen und nichtmaligne Zellen des Tumorstromas synthetisieren und sezernieren das enzymatisch inaktive Proenzym pro-uPA. Proteasen, wie z.B. Plasmin oder die Kathepsine B und L, spalten pro-uPA durch limitierte Proteolyse zur aktiven Serinprotease HMW-uPA (HMW = high molecular weight). Pro-uPA und die aktive Protease HMW-uPA binden an den Zelloberflächenrezeptor uPAR (CD87). Plasmin(ogen) bindet ebenfalls an spezifische Rezeptoren auf der Plasmamembran der Tumorzelle, wodurch eine Fokussierung und Amplifikation der Plasminogenaktivierung in der direkten Umgebung der Tumorzelle erreicht wird. Invasiven Zellen ist somit die Möglichkeit gegeben, die extrazelluläre Matrix abzubauen, ohne sich die für eine gerichtete Bewegung notwendigen Unterlagen durch Proteolyse zu entziehen.

In verschiedenen zellbiologischen Studien konnte gezeigt werden, dass dem zellassoziierten Plasminogenaktivator-System innerhalb der kaskadenartigen Reaktionswege tumorassoziierter Proteolysesysteme ein besonderer Stellenwert zukommt (Wilhelm et al., The Urokinase/Urokinase receptor system: A new target for cancer therapy? In: Schmitt M., Graeff H., Kindermann G. (Hrsg): Prospects in Diagnosis and Treatment of Cancer. International Congress Series, Excerpta Medica 1050, Amsterdam, Elsevier (1994) pp145-156). An Kulturen humaner Kolonkarzinomzellen wurde beobachtet, dass deren Fähigkeit, eine extrazelluläre Matrix zu durchwandern, vom Sättigungsgrad der uPA-Rezeptoren mit aktivem uPA abhängig ist (Hollas et al., Cancer Res. 51 (1991) 3690-3695). Ebenfalls im Zellkulturmodell wurde eine Reduktion des invasiven Potenzials von Zellen beobachtet, wenn die proteolytische Aktivität von uPA durch PAI-1 (Cajot et al., Proc. Natl. Acad. Sci. USA 87 (1990) 6939-6943) oder PAI- 2 (Baker et al., Cancer Res. 50 (1990) 4676-4684) gehemmt wurde. Ein vergleichbarer Effekt wurde bei Hemmung der Bindung von uPA an die Zelloberfläche durch Blockierung des Rezeptors mittels proteolytisch inaktiver uPA-Varianten erzielt (Cohen et al., Blood 78 (1991) 479-487; Kobayashi et al., Br. J. Cancer 67 (1993) 537-544). Auch die Transfektion epidermoider Karzinomzellen mit einem Plasmid, das ein Antisense-Transkript gegen einen Teil von uPAR exprimiert, führte durch Unterdrückung der uPAR-Synthese zur Verringerung der Invasivität dieser Zellen (Kook, EMBO J. 13 (1994) 3983-3991). Gegen uPA und PAI-1 gerichtete Antikörper reduzierten das invasive Potential von Lungenkrebszellen *in vitro* (Liu et al., Int. J. Cancer 60 (1995) 501-506).

Der Einfluss des Plasminogenaktivator-Systems auf den Metastasierungsprozess konnte auch in Tumor-Tiermodellen belegt werden. So wurde die durch menschliche Karzinomzellen verursachte Bildung von Lungenmetastasen in Hühnerembryos durch Zugabe von Antikörpern gegen uPA fast vollständig verhindert (Ossowski und Reich, Cell 35 (1983) 611-619). Metastasierende menschliche Karzinomzellen wurden mit einem Expressionsplasmid transfiziert, das für eine proteolytisch inaktive, aber uPAR-bindende uPA-Mutante kodierte. Im Mausmodell zeigte sich, dass die Karzinomzellen, die inaktives uPA synthetisierten, nach Injektion im Vergleich zu den nicht transfizierten Zellen eine signifikant geringere Anzahl an Metastasen bildeten (Crowley et al., Proc. Natl. Acad. Sci. USA 90 (1993) 5021-5025). Nach Verabreichung von uPA-Antisense Oligonukleotiden wurde darüber hinaus eine Inhibierung der intraperitonealen Ausbreitung von humanen Ovarialkarzinomzellen in Nacktmäusen beobachtet (Wilhelm et al., Clin. Exp. Metast. 13 (1995) 296-302).

In den letzten Jahren wurde die klinische Relevanz von Faktoren des Plasminogenaktivator-Systems (uPA, uPAR, PAI-1 und PAI-2) für die Prognose von Patienten mit soliden malignen Tumoren intensiv untersucht. Dabei erwies sich der uPA Antigengehalt bei verschiedenen Tumoren (z. B. Brust, Eierstock, Magen, Lunge, Niere etc.) sowohl für das rezidiv-freie Überleben, als auch für das Versterben als ein starker Prognosefaktor (siehe beispielsweise Schmitt et al., J. Obstet. Gynaecol. 21 (1995) 151-165; Jaenicke et al., Breast Cancer Res. Treat. 24 (1993) 195-208; Kuhn et al., Gynecol. Oncol. 55 (1994) 401-409; Nekarda et al., Lancet 343 (1994) 117; Pedersen et al., Cancer Res. 54 (1994) 4671-4675). Ebenso korrelieren erhöhte Konzentrationen an uPAR in Lungen- (Pedersen et al., supra) und Brustkrebsgewebe (Duggan et al., Int. J. Cancer 61 (1995) 597-600; Ronne et al., Breast Cancer Res. Treat. 33 (1995) 199-207) sowie bei Magenkrebs, sowohl im Tumorgewebe selbst (Heiss et al., J. Clin.Oncol. 13 (1995) 2084-2093) als auch bei den ins Knochenmark ausgestreuten Tumorzellen (Heiss et al., Nature Medicine 1 (1995) 1035-1039) mit einer schlechten Prognose.

Es wurde auch gefunden, dass an Position 2 mit einem Phenylrest substituierte 3-Amidinophenylalanin-Derivate selektive und *in vivo* wirksame Hemmstoffe von uPA darstellen (EP 1 098 651). Die Verabreichung dieser Verbindungen im Tierexperiment erfolgt in Form von wässrigen Lösungen.

WO 02/074756 und WO 03/103644 offenbaren die Verwendung von weiteren Phenylalanin-basierten Urokinase Inhibitoren als auch die Verwendung von 3-Guanidinophenylalanin-Derivaten als Urokinase-Inhibitoren.

Im Rahmen der ersten klinischen Erprobungen der oben genannten Verbindungen hat sich gezeigt, dass die Verabreichung in Form von wässrigem Mannitol, beispielsweise D-Mannitol, ohne Zusatz von organischen Lösungsmitteln und Propylenglycol/Ethanol sowie Kochsalz enthaltenden Lösungen mit Nachteilen behaftet ist. So lässt sich weder in Kochsalzlösungen noch unter Verwendung des Isotonisierungsmittels Mannitol eine stabile konzentrierte Wirkstoff-Lösung herstellen, die nicht zu Ausfällungen und Niederschlagsbildung neigt. So bildet sich beispielsweise in fünf (5)-prozentigen Mannitollösungen nach längerer Lagerung ein Niederschlag bestehend aus dem zugesetzten Wirkstoff. Als ebenso wenig geeignet haben sich Formulierungen bestehend aus rein organischen Lösungsmitteln erwiesen, da der Wirkstoff in diesen nicht die erforderliche chemische Stabilität aufweist und zur Zersetzung neigt. So setzt nach etwa 1,5 Monaten der Zerfall des Wirkstoffs über Amidbildung zum Ester ein, wobei die Wirkstofflösung unbrauchbar wird.

WO 2004/011004 offenbart einen Ansatz zur Stabilisierung von wässrigen Phenylalanin-basierten Urokinaseinhibitor enthaltenden Lösungen in Form von sogenannten Liposomen, gemischten Micellen bestehend aus verschiedenen Phospholipiden. Diese Stabilisierungsform ist aber nicht für alle Anwendungen ausreichend, insbesondere nach Rekonstitution mit physiologischen Puffern war die chemische Stabilität der liposomalen Formulierung nicht mehr hinreichend gewährleistet.

Vorversuche zur Entwicklung einer neuen Formulierung zeigten eine sehr gute Löslichkeit des Wirkstoffs N$\alpha$-(2,4,6-Triisopropylphenylsulfonyl)-3-amidino-(L)-phenylalanin-4-ethoxy-carbonylpiperazid (WX-UK1) in Polyolen, beispielsweise Diolen, sowie in Mischungen aus Polyol/Alkohol und Wasser (Tabelle 1).

Diese Daten zeigten, dass Mischungen aus Polyol und Alkohol, wie z.B. Propylenglykol (PG) und Ethanol (EtOH), gute Lösungsmittel für eine flüssige Formulierung darstellen. Zusätzlich sind beide Lösungsmittel geeignet für eine parenterale Gabe von Wirkstoffen.

Die Lagerung von verschiedenen Formulierungen, beispielsweise

a) WX-UK1 60mg/ml in PG/EtOH/Wasser 40/10/50
b) WX-UK1 50mg/ml in PG/EtOH/Wasser 40/10/50
c) WX-UK1 40mg/ml in PG/EtOH/Wasser 40/10/50
d) WX-UK1 20mg/ml in PG/EtOH/Wasser 10/10/80
e) WX-UK1 4mg/ml in Wasser (Kontrolle)
f) WX-UK1 4mg/ml in 5 % D-Mannitol (Kontrolle)

bei zwei bis acht Grad Celsius (2-8 °C) haben ergeben, dass sich in Formulierung (e) und (f) schon nach wenigen Stunden ein nadelartiger kristalliner Niederschlag bildet. Nach vier (4) Tagen bei zwei bis acht Grad Celsius (2-8 °C) bildet sich auch in der Formulierung (d) ein ähnlich aussehender Niederschlag.

In den Formulierungen (a) und (b) zeigt sich nach 16 bzw. nach 22 Tagen Lagerung bei zwei bis acht Grad Celsius (2-8 °C) kein Niederschlag.

Stabilitätsstudien bei 2-8 °C, 25 °C/60 % RF (relative Luftfeuchtigkeit) und 40 °C/75 % RF zeigt die Formulierung (c) unter 40 °C Bedingungen bereits nach 6 Wochen ca. 4 %, nach 8 Wochen ca. 23 % und nach 12 Wochen ca. 38 % Verunreinigungen, im Vergleich zu ca. 0,5 % zu Beginn der Untersuchung. Gleichzeitig stieg der pH-Wert der Formulierung über einen Zeitraum von 12 Wochen von 5,1 auf 8,7 an (Abbildung 1).

Der Anstieg des pH-Wertes ist vermutlich auf den Zerfallsprozess von WX-UK1 zurückzuführen. Abbildung 2 zeigt eine mögliche Zerfallsreaktion des Wirkstoffs WX-UK1 in wässrigen Medien auf: WX-UK1 zerfällt im ersten Schritt zum entsprechenden WX-UK1-Amid, wobei Ammoniak freigesetzt wird, das allerdings durch das Vorliegen von WX-UK1 als Hydrochlorid in Form von Ammoniumchlorid abgefangen wird. Im zweiten Zerfallsschritt reagiert das WX-UK1-Amid mit Alkohol unter der Freisetzung weiteren Ammoniaks zum entsprechenden WX-UK1-Ester. Die Freisetzung des Ammoniaks ist vermutlich für den Anstieg des pH-Werts verantwortlich.

Aufgrund der Erkenntnisse über den Zerfallsprozess wurden bevorzugt wasserfreie Formulierungen in Betracht gezogen, um die wasserbedingte Zersetzung des Wirkstoffs zu vermeiden. Die relativ hohe Viskosität der rein organischen Lösungsmittel stellt jedoch eine Erschwernis hinsichtlich der Handhabbarkeit der zähflüssigen Konzentrate im täglichen Klinikalltag dar. Zudem führt die stark hygroskopische Eigenschaft der Polyole zur Aufnahme von Wasser, was den Zerfallsprozess des Wirkstoffs wieder in Gang setzt. Auch die Pufferung mit organischen Puffern, wie z.B. Triethanolamin/HCl, Piperazin/HCl, Propionsöure/Propionat, die nicht alle physiologisch verträglich sind, gestaltet sich schwierig.

Versuche zur Stabilisierung von wässrigen Lösungen durch Zusatz oberflächenaktiver Mittel, wie etwa Pluronic F68 oder Tween 80, bzw. Stabilisatoren, wie Humanserumalbumin, waren erfolglos. Zusätze von Cosolventien, wie

Polyethylenglykolen, sowie Formulierung des Wirkstoffs in gemischten Micellen, die das Gallensalz Glycocholatmonohdyrat und das Phospholipid Ei-Phosphatidylcholin enthielten, sorgte ebenfalls nicht für eine ausreichende Stabilität.

Es bestand daher das Bedürfnis, neue pharmazeutische Formulierungen für Wirkstoffe mit Amidino- und/oder Guanidinogruppen zu entwickeln, die einerseits physikalisch wie chemisch stabil sind und sich portioniert oder/und als Konzentrat gut handhaben und einlagern lassen, um bei Bedarf stabile pharmazeutische Präparate, z.B. mit geeigneten Isotonisierungsmitteln stabile physiologische Infusionslösungen zuzubereiten, die verträglich sind und eine hohe Wirksamkeit besitzen.

Diese Aufgabe wird erfindungsgemäß gelöst durch eine pharmazeutische Formulierung gemäß Anspruch 1, umfassend (i) ein Amidino-, Hydroxyamidino-, Guanidino- und/oder Hydroxyguanidinophenylalanin-Derivat als Wirkstoff, (ii) ein Gemisch aus einem Polyol und einem Alkohol und (iii) eine wässrige Phase, umfassend einen Puffer.

Als Wirkstoff wird vorzugsweise ein als Serinprotease-Inhibitor, insbesondere Urokinase-Inhibitor wirksames Phenylalanin-Derivat verwendet. Bevorzugte Wirkstoffe sind die in EP-A-1 098 651, WO 02/074756 und WO 03/103644 offenbarten Amidinophenylalanin- oder Guanidinophenylalaninverbindungen. Ebenfalls bevorzugt sind Hydroxyamidinophenylalanin- oder Hydroxyguanidinophenylalaninverbindungen, wie in PCT/EP2004/005682 offenbart. Insbesondere sind als Wirkstoffe der erfindungsgemäßen pharmazeutischen Formulierung von 3-Amidinophenylalanin oder 3-Guanidinophenylalanin abgeleitete neue Urokinase-Inhibitoren der allgemeinen Formel I geeignet,

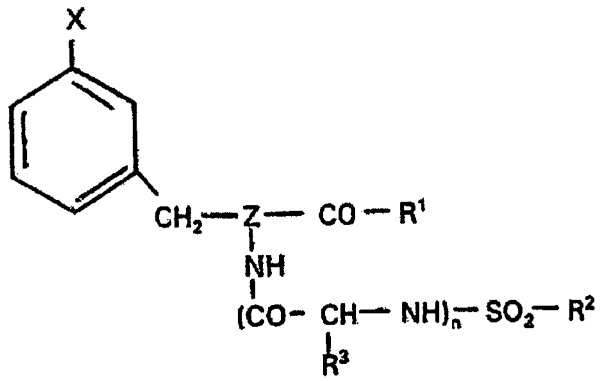

(I)

die als Racemate sowie als L- bzw. D-konfigurierte Verbindungen vorliegen und in denen

X eine Amidino- oder Guanidinogruppe oder Hydroxyamidino- oder Hydroxyguanidinogruppe ist,

$R^1$ (a) OH oder $OR^4$ ist, wobei $R^4$ ein gegebenenfalls z.B. mit Hydroxyl, Carboxyl, Sulfonyl, Nitro, Cyano, Oxo oder/und Halogen substituiertes, verzweigtes oder unverzweigtes $C_1$-$C_8$-Alkyl, $C_3$-$C_8$-Cycloalkyl oder Aralkyl, z. B. Benzyl oder Phenylethyl, ist,

(b) eine Gruppe der Formel

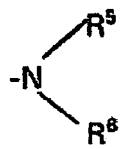

darstellt, in welcher $R^5$ und $R^6$ beliebige mit der Gesamtstruktur kompatible Reste sind, wobei insbesondere

(i) $R^5$ und $R^6$ H sind,
(ii) $R^5$ H ist und $R^6$ ein gegebenenfalls z.B. mit Hydroxyl, Carboxyl, Sulfonyl, Nitro, Cyano, Oxo oder/und Halogen substituiertes verzweigtes oder unverzweigtes $C_1$-$C_8$-Alkyl, Aralkyl, z.B. Benzyl oder Phenylethyl, oder $C_5$-$C_8$-Cycloalkyl ist,
(iii) $R^5$ und $R^6$ jeweils unabhängig ein gegebenenfalls z.B. mit Hydroxyl oder/und Halogen substituiertes, unverzweigtes oder verzweigtes $C_1$-$C_4$-Alkyl sind oder
(iv) $R^5$ H ist und $R^6$ $-NH_2$ oder eine insbesondere mit Aryl oder Heteroaryl substituierte Aminogruppe ist,

(v) $R^5$ H oder ein gegebenenfalls z.B. mit Hydroxyl oder/und Halogen substituiertes, unverzweigtes oder verzweigtes $C_1$-$C_4$-Alkyl ist und $R^6$ der Rest einer Aminosäure, z.B. einer $\alpha$, $\beta$- oder $\omega$-Aminocarbon- oder Aminosulfonsäure, oder der Rest eines Peptids z. B. mit einer Länge bis zu 50 Aminosäuren oder eines Polypeptids z.B. mit einer Länge von mehr als 50 Aminosäuren bis 1.000 Aminosäuren ist,

(c) eine Gruppe der Formel

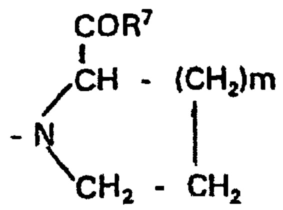

darstellt, in welcher m die Zahl 1 oder 2 bezeichnet, und in welcher eine oder mehrere der Methylengruppen gegebenenfalls z.B. mit einem Hydroxyl-, Carboxyl-, $C_1$-$C_4$-Alkyl- oder Aralkylrest, z.B. Benzyl oder Phenylethyl, substituiert sind, wobei die Gruppe (c) racemisch oder D- bzw. L-konfiguriert ist, und $R^7$ die Bedeutung von $R^1$ in den Ziffern (a), (b) und (f) aufweist,

(d) eine Gruppe der Formel

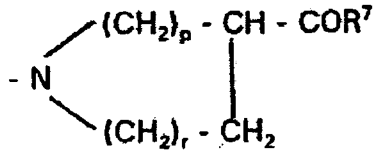

darstellt, in welcher p = r = 1, p = 1 und r = 2 oder p = 2 und r = 1 sind und in welcher eine oder mehrere der Methylengruppen gegebenenfalls z.B. mit einem Hydroxyl-, Carboxyl-, $C_1$-$C_4$-Alkyl- oder Aralkylrest, z.B. Benzyl oder Phenylethyl, substituiert sind, und $R^7$ die Bedeutung von $R^1$ in Ziffer (a), (b) und (f) aufweist,

(e) eine Piperidylgruppe darstellt, die gegebenenfalls in einer der Stellungen 2, 3 und 4 z.B. mit einem $C_1$-$C_4$-Alkyl-, $C_1$-$C_3$-Alkoxy- oder Hydroxylrest substituiert ist, wobei gegebenenfalls an die heterocycloaliphatischen Ringe der Formeln (c), (d), (e) ein weiterer aromatischer oder cycloaliphatischer Ring, vorzugsweise Phenyl oder Cyclohexyl, in 2,3- oder 3,4-Stellung, bezogen auf das Heteroatom, ankondensiert ist,

(f) eine Gruppe der Formel

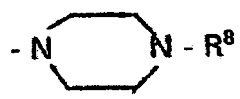

darstellt, in welcher $R^8$

(i) einen gegebenenfalls z.B. mit $C_1$-$C_6$-Alkyl, $C_1$-$C_3$-Alkoxy, Hydroxyl, Carboxyl, Sulfonyl, Nitro, Cyano, Oxo oder/und Halogen substituierten $C_1$-$C_6$-Alkylrest, wie z.B. Ethoxycarbonyl, oder Arylrest, wie z.B. Phenyl, p-Halogenphenyl, Naphthyl,
(ii) einen gesättigten oder ungesättigten, verzweigten oder unverzweigten $C_1$-$C_6$-Alkoxyrest oder
(iii) einen gegebenenfalls z.B. mit $C_1$-$C_6$-Alkyl, $C_1$-$C_3$-Alkoxy, Hydroxyl, Carboxyl, Sulfonyl, Nitro, Cyano, Oxo oder/und Halogen substituierten Phenoxy- bzw. Benzyloxycarbonylrest bedeutet,

(g) einen Acylrest der Formel-COX darstellt, wobei X

(i) H, einen gegebenenfalls z.B. mit Hydroxyl, Carboxyl, Sulfonyl, Nitro, Cyano, Oxo oder/und Halogen substituierten, unverzweigten oder verzweigten Alkylrest, vorzugsweise einen $C_1$-$C_6$-Alkylrest, insbesondere Methyl,
(ii) einen gegebenenfalls z.B. mit $C_1$-$C_6$-Alkyl, $C_1$-$C_3$-Alkoxy, Hydroxyl, Carboxyl, Sulfonyl, Nitro, Cyano, Oxo oder/und Halogen substituierten Aryl- oder Heteroarylrest, wie z.B. Phenyl, p-Halogenphenyl, Thienyl oder
(iii) einen gegebenenfalls z.B. mit Hydroxyl, Carboxyl, Sulfonyl, Nitro, Cyano, Oxo oder/und Halogen substituierten Cycloalkylrest, vorzugsweise einen $C_3$-$C_{10}$-Cycloalkylrest bedeutet,

(h) einen Aralkylrest, z.B. Benzyl oder Phenylethyl, darstellt, in dem der aromatische Rest gegebenenfalls z.B. mit einem Halogenatom, einer $C_1$-$C_6$-Alkyl-, $C_1$-$C_3$-Alkoxy-, Hydroxy-, Cyano-, Carboxyl-, Sulfonyl- oder Nitrogruppe substituiert ist,

(i) einen Carbonsäureamidrest der Formel -CONR'R", einen Thiocarbonsäureamidrest -CSNR'R" oder einen Essigsäureamidrest -$CH_2$-CONR'R" darstellt, wobei

(i) R' und R" H sind,
(ii) R' und R" jeweils unabhängig $C_1$-$C_4$-Alkyl sind,
(iii) R' H ist und R" $C_1$-$C_4$-Alkyl ist,
(iv) R' H ist und R" Aryl, z. B. Phenyl, ist oder
(v) R' und R" mit dem Stickstoffatom einen heterocycloaliphatischen Ring mit 5-7 Ringgliedern, der ein weiteres Heteroatom, z.B. N, O oder/und S tragen kann, bilden,

(j) einen $SO_2$-Y-Rest darstellt, in dem Y

(i) ein gegebenenfalls z.B. mit Hydroxyl, Carboxyl, Sulfonyl, Nitro, Cyano, Oxo oder/und Halogen substituiertes $C_1$-$C_8$-Alkyl, vorzugsweise Methyl, Trifluormethyl, Trichlormethyl,
(ii) ein gegebenenfalls z. B. mit $C_1$-$C_6$-Alkyl, $C_1$-$C_3$-Alkoxy, Hydroxyl, Carboxyl, Sulfonyl, Nitro, Cyano, Oxo oder/und Halogen substituiertes Aryl oder Heteroaryl, wie z.B. Phenyl, 4-Methylphenyl, 2,4,6-Trimethylphenyl, 2,4,6-Triisopropylphenyl, 4-Methoxy-2, 3,6-Trimethylphenyl, 2,2-Dimethyl-6-methoxychromanyl, 2,2,5,7,8-Pentamethylchromanyl, Anthrachinonyl, Naphthyl oder Chinolyl, bzw. O-Aryl, vorzugsweise O-Phenyl, oder O-Heteroaryl oder
(iii) -NR'R" ist, wobei R' und R" jeweils unabhängig H oder C1-C3-Alkyl bedeuten,

(k) einen cycloaliphatischen Ring mit 5 bis 8 C-Atomen darstellt, der gegebenenfalls z.B. mit einer $C_1$-$C_6$-Alkyl-, $C_1$-$C_3$-Alkoxy-, Halogen-, Hydroxyl- oder/und Oxogruppe substituiert ist,

(l) einen gegebenenfalls z.B. mit $C_1$-$C_6$-Alkyl, $C_1$-$C_3$-Alkoxy, Hydroxyl, Carboxyl, Sulfonyl, Nitro, Cyano, Oxo oder/und Halogen substituierten Heteroarylrest, wie z.B. Pyridyl oder Pyrimidyl, oder heterocycloaliphatischen Rest, beispielsweise N-Methylpiperidyl darstellt,

(m) einen funktionalisierten Alkylrest der Formel $-(CH_{2})_{n}-X$ darstellt, wobei die Alkylkette unverzweigt oder verzweigt ist, n = 1 bis 8 bedeutet und der funktionelle Rest X

(i) eine Hydroxylgruppe darstellt, deren H-Atom gegebenenfalls durch eine $C_1$-$C_4$-Alkyl-, Aralkyl-, z.B. Benzyl oder Phenylethyl, Aryl-, z.B. Phenyl, $C_1$-$C_4$-Hydroxyalkyl- oder Acylgruppe, CO-Alkyl ($C_1$-$C_6$), substituiert ist,
(ii) ein Halogenatom bedeutet,
(iii) eine tertiäre Aminogruppe der Formel -$N(Alk)_2$ darstellt, wobei die Alkylgruppen 1 bis 3 C-Atome sowie vorzugsweise die gleiche Bedeutung besitzen und das Stickstoffatom gegebenenfalls einem heterocycloaliphatischen Ring mit 5-7 Ringgliedern, der ein weiteres Heteroatom, z.B. N, O oder/und S tragen kann, angehört,

$R^2$ einen gegebenenfalls z.B. mit $C_1$-$C_6$-Alkyl, $C_1$-$C_3$-Alkoxy, Hydroxyl, Carboxyl, Sulfonyl, Nitro, Cyano, Oxo oder/und Halogen substituierten Phenylrest, wie beispielsweise Phenyl, 4-Methylphenyl, 2,4,6-Tri- methylphenyl, 2,4,6-Triisopropylphenyl, 4-Methoxy-2,3,6-trimethyl- phenyl darstellt,

$R^3$ H oder verzweigtes bzw. unverzweigtes $C_1$-$C_4$-Alkyl ist und n 0 oder 1 bedeutet,

Z N oder $CR^9$ bedeutet, wobei $R^9$ H oder verzweigtes oder unverzweigtes $C_1$-$C_4$-Alkyl ist.

Die Verbindungen können auch als Salze, vorzugsweise als physiologisch verträgliche Säuresalze, z.B. als Salze von Mineralsäuren, besonders bevorzugt als Hydrochloride, Hydrogensulfate, Sulfate oder als Salze von geeigneten organischen Säuren vorliegen.

Von den in den allgemeinen Ansprüchen definierten Verbindungen sind solche, bei denen $R^1$ einer Gruppe der Formeln (b), (d) und (f) entspricht, $R^2$ einen einfach, zweifach oder dreifach alkylsubstituierten Phenylrest, insbesondere einen 2,4,6-substituierten Phenylrest, z.B. einen 2,4,6-Triisopropylphenyl-Rest darstellt, und n = 0 ist, von besonderer Bedeutung. Weiterhin bevorzugt sind Verbindungen, bei denen Z CH oder N ist.

Besonders bevorzugt handelt es sich bei der Verbindung der Formel (I) um Na-(2,4,6-Triisopropylphenylsulfonyl)-3-amidino-(D,L)-phenylalanin-4-ethoxycarbonylpiperazid, Nα-(2,4,6-Triisopropylphenylsulfonyl)-3-guanidino-(D, L)-phenylalanin-4-ethoxycarbonylpiperazid bzw. um das L-Entantiomere davon oder um ein pharmazeutisch verträgliches Salz dieser Verbindungen.

Wie oben erwähnt, sind auch entsprechende Hydroxyverbindungen der Amidino- und Guanidinophenylalanin-Derivate als Wirkstoffe geeignet, z.B. solche, wie in PCT/EP2004/005682 offenbart, insbesondere Verbindungen der allgemeinen Formel II oder/und III

II

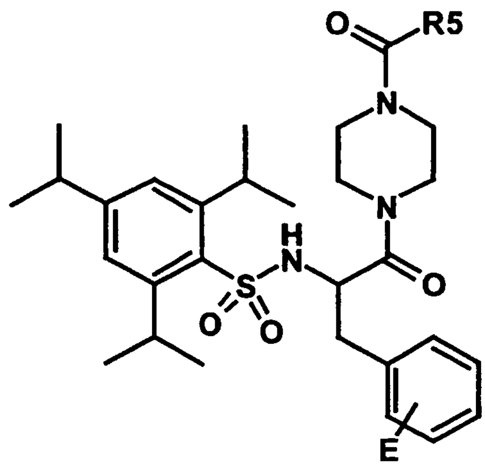

III

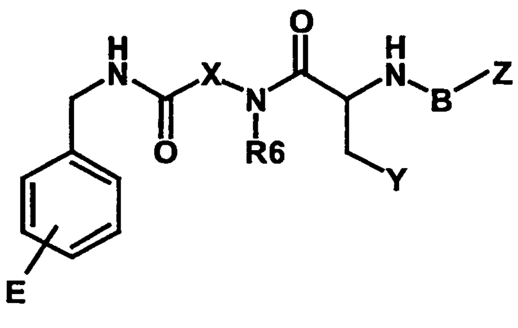

worin

E eine Gruppe aus

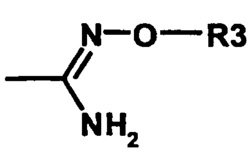 (Am) 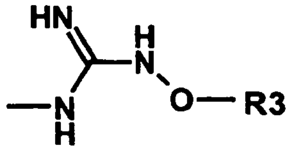 (Gua) bedeutet,

- B -$SO_2$- oder -CO- bedeutet,
- X -$NR^1$ oder -$CHR^1$ bedeutet,
- Z -$R^4$, -$OR^4$ oder -NH-$R^4$ bedeutet,
- Y -$OR^2$ oder -$NHR^2$ bedeutet,
- $R^1$ jeweils unabhängig -H, -$C_1$-$C_6$-Alkyl, -$C_2$-$C_6$-Alkenyl oder -$C_2$- $C_6$-Alkinyl unsubstituiert oder substituiert bedeutet,
- $R^2$ -H, -$OR^1$, -$COR^1$, -$COOR^1$ oder -$CON(R^1)_2$ bedeutet,
- $R^3$ -H, -$C_1$-$C_6$-Alkyl, -$C_2$-$C_6$-Alkenyl oder -$C_2$-$C_6$-Alkinyl, unsubstituiert oder substituiert oder -$COR^6$ oder -$COOR^6$ oder einen Oligo- oder Poylalkylenoxyrest mit z.B. 2-50 -$C_2$-$C_4$- Alkylenoxy, z.B. Ethylenoxyresten bedeutet,
- $R^4$ -H, -$C_1$-$C_6$-Alkyl, -$C_2$-$C_6$-Alkenyl oder -$C_2$-$C_6$-Alkinyl unsubstituiert oder substituiert oder einen cyclischen Rest bedeutet und
- $R^5$ -$OR^6$, -$N(R^6)_2$, -$C_1$-$C_6$-Alkyl, -$C_2$-$C_6$-Alkenyl oder -$C_2$-$C_6$-Alkinyl unsubstituiert oder substituiert bedeutet, und
- $R^6$ -H, -$C_1$-$C_6$-Alkyl, -$C_2$-$C_6$-Alkenyl oder -$C_2$-$C_6$-Alkinyl unsubstituiert oder substituiert oder einen cyclischen Rest bedeutet,

wobei jeder cyclische Rest einen oder mehrere Substituenten, z.B. ausgewählt aus -$C_1$-$C_3$-Alkyl, -$OR^6$ (z.B. -OH oder -$C_1$-$C_3$-Alkoxy), Halogen, =O -$NO_2$, -CN, -$COOR^6$, -$N(R^6)_2$, -$NR^6COR^6$, -$NR^6CON(R^6)_2$ und -$OCOR^6$ tragen kann, und wobei jedes Alkyl, Alkenyl und Alkinyl geradkettig oder verzweigt sein kann und einen oder mehrere Substituenten, z.B. ausgewählt aus Halogen (F, Cl, Br, I), -$OR^6$, -$OCOR^6$, -$N(R^6)_2$, -$NR^6COR^6$, $COOR^6$, -$NR^6COR^6$ oder einem cyclischen Rest, tragen kann
oder Salze dieser Verbindungen sowie gegebenenfalls pharmazeutisch übliche Träger, Verdünnungs- oder/und Hilfsmittel.

Bevorzugt sind Verbindungen der allgemeinen Formel IV

IV

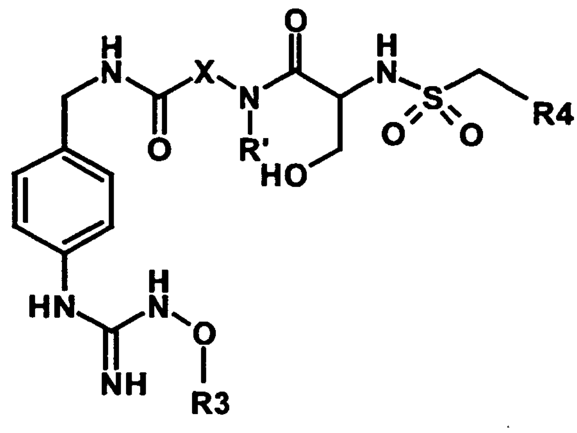

worin

X, $R^1$, $R^3$,$R^4$ und $R^6$ wie oben definiert sind,

oder deren Salze.

Die Gruppe E befindet sich vorzugsweise in para-Position des Phenylringes bei den Verbindungen I und II. Besonders bevorzugt sind Verbindungen der allgemeinen Formel I, worin E Am ist.

Die erfindungsgemäßen Verbindungen weisen eine modifizierte Amidino- oder Guanidinofunktion E, vorzugsweise eine Hydroxyguanidino- oder Hydroxyamidinofunktion auf. Derartige Modifikationen waren lediglich als Synthesezwischenprodukte bei der Herstellung von Urokinase-Inhibitoren des Guanidino- oder Amidinotyps bekannt. Eine pharmazeutische Wirksamkeit wurde bisher nicht vermutet.

Die Verbindungen können als Salze, vorzugsweise als physiologisch verträgliche Säuresalze, z.B. Salze von Mineralsäuren, besonders bevorzugt als Hydrochloride oder Hydrogensulfate, oder als Salze von geeigneten organischen Säuren, z.B. von organischen Carbon- oder Sulfonsäuren, wie etwa Tartrate, Mesylate oder Besylate, vorliegen. Besonders bevorzugt sind die Hydrogensulfate. Die Verbindungen können als optisch reine Verbindungen oder als Gemische von Enantiomeren oder/und Diastereomeren vorliegen.

Cyclische Reste können einen oder mehrere gesättigte, ungesättigte oder aromatische Ringe enthalten. Bevorzugte Beispiele für cyclische Reste sind Cycloalkylreste, Arylreste, Heteroarylreste und bicyclische Reste. Besonders bevorzugt sind mono- oder bicyclische Reste. Die cyclischen Reste enthalten vorzugsweise 4 bis 30, insbesondere 5-10 Kohlenstoff- und Heteroatome als Ringatome, sowie gegebenenfalls einen oder mehrere Substituenten wie zuvor angegeben. Heterocyclische Systeme enthalten bevorzugt ein oder mehrere O-, S- oder/und N-Atome. Bevorzugte bicyclische Ringsysteme sind solche mit einem -CO-Rest.

Alkyl-, Alkenyl- und Alkinylgruppen enthalten vorzugsweise bis zu 4 Kohlenstoffatome. $R^1$ ist bevorzugt H oder ein gegebenenfalls substituierter $C_1$-$C_4$-Alkylrest, z.B. $-CH_3$ oder ein $C_1$-$C_6$-Alkyl-arylrest, so dass -CO-X-$NR^1$ z.B. einen Glycyl-, Alanyl-, Phenylalanyl- oder Homophenylalanylrest darstellen kann. $R^2$ ist besonders bevorzugt H oder ein $C_1$-$C_3$-Alkylrest, so dass Y z.B. einen OH- oder O-$C_1$-$C_3$-Alkylrest darstellen kann. $R^3$ ist besonders bevorzugt H. In den Verbindungen I bedeutet $R^5$ bevorzugt -$NHR^6$, besonders bevorzugt -NH($C_1$-$C_5$)-Alkyl, unsubstituiert oder substituiert, z.B. $-NHC_2H_5$ oder -$OR^6$, besonders bevorzugt -O($C_1$-$C_3$)Alkyl, unsubstituiert oder substituiert, z.B. Ethyloxy oder Benzyloxy, oder -O-Aryl, z.B. Phenyloxy. In den Verbindungen II und III ist $R^6$ vorzugsweise -H oder $C_1$-$C_3$-Alkyl.

Bevorzugt sind Verbindungen, worin das Strukturelement Z für $R^4$ steht, worin R4 einen Alkylrest mit einem cyclischen Substituenten, z.B. einem gegebenenfalls substituierten Phenylrest oder einem bicyclischen Rest, wie etwa

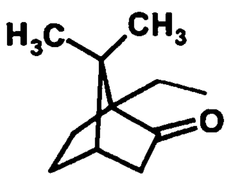

oder

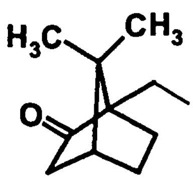

bedeutet.

Besonders bevorzugte Verbindungen sind solche, worin $R^4$ einen substituierten oder unsubstituierten $C_1$-$C_3$-Alkyl-Aryl-Rest bedeutet, z.B. einen Benzylrest, der gegebenenfalls in meta- oder para-Position mit Halogen oder/und $-NO_2$ substituiert sein kann, wobei das Halogen ausgewählt wird aus F, Cl, Br und I, besonders bevorzugt Cl und Br.

Am meisten bevorzugt sind die Verbindungen

N-α-(2,4,6-Triisopropylphenyl-sulfonyl)-3-hydroxyamidino-(L)-phenylalanin-4-ethoxy-carbonylpiperazid (WX- 671),
N-α-(2,4,6-Triisopropyl-phenylsulfonyl)-3-hydroxyamidino-(D)-phenyl-alanin-4-ethoxycarbonyl-piperazid,
N-α-(2,4,6-Triisopropylphenylsulfonyl)-3-hydroxyamidino-(D,L)-phenylalanin-4-ethoxycarbonylpiperazid,
N-α-(2,4,6-Triisopropylphenylsulfonyl)-3-hydroxyguanidino-(L)-phenylalanin-4-ethoxy- carbonylpiperazid (WX-683),
N-α-(2,4,6-Triisopropylphenylsulfonyl)-3-hydroxyguanidino-(D)-phenylalanin-4-ethoxycarbonylpiperazid,
N-α-(2,4,6-Triisopropylphenyl-sulfonyl)-3-hydroxyguanidino-(D,L)-phenylalanin-4-ethoxy-carbonyl-piperazid,
N-α-(2,4,6-Triisopropylphenylsulfonyl)-3-hydroxy-guanidino-(L)-phenylalanin-4-ethylaminocarbonylpiperazid (WX-685),
N-α-(2,4,6-Triisopropylphenylsulfonyl)-3-hydroxyguanidino-(D)-phenylalanin-4-ethyl-aminocarbonylpiperazid,
N-α-(2,4,6-Triisopropylphenylsulfonyl)-3-hydroxyguanidino-(D,L)-phenylalanin-4-ethylaminocarbonylpiperazid,
Benzylsulfonyl-(D)-Ser-Gly-(4-hydroxyguanidinobenzyl)amid (WX-678),
4-Chlorbenzylsulfonyl-(D)-Ser-N-Me-Ala-(4-hydroxyguanidinobenzyl)amid, 4-Chlorbenzylsulfonyl-(D)-Ser-Gly-(4-hydroxyguanidinobenzyl)amid,
Benzylsulfonyl-(D)-Ser-N-Me-Gly-(4-hydroxyguanidinobenzyl)amid,
4-Chlorbenzylsulfonyl-(D)-Ser-Ala-(4-hydroxyguanidinobenzyl)amid
sowie deren Salze, z.B. die Hydrogensulfate, wie etwa WX-671 · $HSO_4$.

Die erfindungsgemäßen Formulierungen enthalten eine therapeutisch wirksame Menge des Phenylalaninderivat-basierten Wirkstoffs, eine physiologisch verträglichen Menge des Alkohols und Polyols, eine wässrige Phase mit Pufferbestandteilen sowie gegebenenfalls Isotonisierungsmittel und weitere Hilfsstoffe einzeln oder in Mischungen oder

Kombinationen davon.

Die erfindungsgemäßen Formulierungen enthalten den Wirkstoff vorzugsweise in einem Gewichtsanteil von 0,5 bis 10 %, bevorzugt von 1 bis 9 %, besonders bevorzugt von 2 bis 5 %, bezogen auf das Gesamtgewicht der Formulierung.

Der Wirkstoff ist vorzugsweise in einer Konzentration von bis zu 100 mg/ml, vorzugsweise bis zu 80 mg/ml, vorzugsweise bis zu 60 mg/ml, vorzugsweise bis zu 50 mg/ml, stärker bevorzugt bis zu 40 mg/ml, noch stärker bevorzugt etwa 30 mg/ml, noch stärker bevorzugt etwa 20 mg/ml, noch stärker bevorzugt etwa 10 mg/ml, noch stärker bevorzugt etwa 4 mg/ml, noch stärker bevorzugt bis etwa 1 mg/ml, vorzugsweise bis etwa 0,1 mg/ml vorhanden. Die Formulierung kann gegebenenfalls vor Anwendung weiter verdünnt werden.

Der Alkohol oder das Polyol im Sinne dieser Erfindung umfassen physiologisch verträgliche ein- und mehrwertige Alkohole. Unter einem Polyol wird hierin ein mehrwertiger Alkohol verstanden. Insbesondere kann dies ein zweiwertiger Alkohol (Diol) oder ein dreiwertiger Alkohol (Triol) sein, oder auch ein mehrwertiger Alkohol.

Als einwertiger Alkohol ist beispielsweise Ethanol bevorzugt. Es können aber auch weitere physiologisch verträgliche Alkohole verwendet werden.

Als Polyole kommen insbesondere physiologisch verträgliche Diole und Triole in Frage, bevorzugt ist als Triol z.B. Glycerin. Auch Glycole sind erfindungsgemäß geeignet. Beispiele für Glycole sind Glycol, Propylenglycol, Polyethylenglycol.

Der Alkohol und das Polyol sind in der erfindungsgemäßen pharmazeutischen Formulierung vorzugsweise in einer solchen Menge vorhanden, dass diese Komponente etwa 20-60 %, vorzugsweise 40-60 %, stärker bevorzugt 45-55 %, am meisten bevorzugt etwa 50 % bezogen auf das Volumen der gesamten Formulierung ausmacht.

Besonders bevorzugt ist ein Gemisch aus Polyol und Alkohol. Das Verhältnis von Polyol:Alkohol ist hierbei bevorzugt von 2:1 bis 10:1, stärker bevorzugt 3:1 bis 8:1, noch stärker bevorzugt 4:1 bis 6:1 und am stärksten bevorzugt 4:1.

Bei dem Gemisch handelt es sich vorzugsweise um ein Gemisch aus einem Glycol und Ethanol. Besonders bevorzugt ist ein Gemisch aus Propylenglycol und Ethanol sowie Polyethylenglycol und Ethanol.

Die wässrige Phase, umfassend einen Puffer, ist vorzugsweise ausgewählt aus einer Gruppe der physiologisch verträglichen Puffer, insbesondere Acetatpuffer, Citratpuffer, Phosphatpuffer und dgl. vorzugsweise handelt es sich bei der Pufferkomponente um einen Natriumacetatpuffer. Es sind jedoch auch andere Acetatpuffer geeignet, z.B. Kaliumacetatpuffer, Calciumacetatpuffer. Der Fachmann ist in der Lage, aus den physiologisch verträglichen Puffern, insbesondere Acetatpuffern, einen geeigneten Puffer auszuwählen.

Die wässrige Phase liegt vorzugsweise in einer Menge bis zu 70 % bezogen auf das Volumen der gesamten Formulierung vor, vorzugsweise bis zu 60 %, stärker bevorzugt etwa bis 50 %. Selbstverständlich kann die pharmazeutische Formulierung, falls erforderlich, vor der Anwendung verdünnt werden. Vorzugsweise wird die Formulierung direkt vor Anwendung verdünnt, vorzugsweise mit einem Isotonisierungsmittel oder einer isotonischen Flüssigkeit, so dass vorzugsweise ein zur Infusion oder Injektion geeignete isotonische Lösung erzeugt wird.

Der Puffer liegt vorzugsweise in einer Konzentration von bis zu 1000 mM, vorzugsweise bis zu 500 mM, stärker bevorzugt bis zu 250 mM, stärker bevorzugt bis zu 200 mm, noch stärker bevorzugt etwa 100 mM.

Zusätzlich kann die die erfindungsgemäße Formulierung ein Isotonisierungsmittel und/oder weitere Hilfsstoffe umfassen, die dem Fachmann geläufig sind.

Das Isotonisierungsmittel ist vorzugsweise ein Zucker oder vorzugsweise ausgewählt aus z.B. Glucose, Ribose, Saccharose, Sorbitol, Mannitol, Lactose, Dextrose, Trehalose, Glycerin und Gemischen davon. Vorzugsweise liegt das Isotonisierungsmittel in Form einer Lösung vor. Vorzugsweise wird als Isotonisierungsmittel eine etwa 1 bis 10-prozentige, vorzugsweise 2 bis 7-prozentige, besonders bevorzugt 5-prozentige Lösung verwendet. Besonders bevorzugt ist eine Glucoselösung.

Die erfindungsgemäße Formulierung kann weiterhin Hilfsstoffe enthalten, die der Fachmann leicht ermitteln kann.

Weiterhin ist bevorzugt, dass die erfindungsgemäße Formulierung - zumindest deren wässrige Komponente - einen pH-Wert im Bereich von 3,5 bis 9,0 aufweist, bevorzugt einen pH-Wert im Bereich von 4 bis 7, und besonders bevorzugt einen pH-Wert im Bereich 4,5 bis 5,5.

Die erfindungsgemäße Formulierung kann für verschiedene Verabreichungsrouten verwendet werden, z.B. als flüssige Formulierung, parenteral, als Infusion, intramuskulär, intravenös, subkutan etc.

Vorzugsweise wird die Formulierung intravenös oder intramuskulär verabreicht. Die hierzu geeigneten Hilfsstoffe können vom Fachmann auf dem Gebiet leicht ermittelt werden.

Die erfindungsgemäße Formulierung kann gegebenenfalls in Kombination mit anderen Wirkstoffen, z.B. zytostatischen oder zytotoxischen Mitteln, beispielsweise Doxorubicin, cis-Platin, 5-Fluor-Uracil oder Antikörpern und Peptiden eingesetzt werden.

Die erfindungsgemäße Formulierung kann zur parenteralen Applikation, z.B. zur intravenösen oder intramuskulären Injektion oder/und zur Infusion eingesetzt werden. Die Tagesdosis ist vorzugsweise 5-250 mg, besonders bevorzugt von 20-120 mg bei subkutaner oder intramuskulärer Verabreichung und von 10-500 mg, besonders bevorzugt von 50-250 mg bei intravenöser Verabreichung, jeweils bezogen auf ein mittleres Körpergewicht von 70 kg. Die Verabreichung erfolgt vorzugsweise einmal täglich bis einmal wöchentlich.

Ein weiterer Gegenstand der Erfindung betrifft ein Konzentrat bestehend aus einer erfindungsgemäßen Formulierung, wobei der Wirkstoff in einer Konzentration von bis zu 100 mg/ml, vorzugsweise bis zu 80 mg/ml, stärker bevorzugt bis zu 50 mg/ml, noch stärker bevorzugt etwa 40 mg/ml vorliegt. Der Puffer liegt vorzugsweise in einer Konzentration von bis zu 1000 mM vor, vorzugsweise bis zu 500 mM, stärker bevorzugt bis zu 250 mM, noch stärker bevorzugt etwa 100 mM.

Besonders bevorzugt ist ein Konzentrat, bei dem die Konzentration des Wirkstoffs 40 mg/ml und die Konzentration des Puffers 100 mM beträgt.

Die erfindungsgemäßen Konzentrate und Formulierungen können ohne großen Reinheits- und Wirkstoffverlust längere Zeit, typischerweise bei zwei bis acht (2-8) °C aber auch bei Einlagerung bei erhöhter Temperatur, z.B. bei 40° C aufbewahrt werden.

Die Wirkstoffverbindungen sind zur Bekämpfung von Krankheiten geeignet, die mit einer pathologischen Überexpression von uPA oder/und Urokinase-Plasminogenaktivatorrezeptor (uPAR) assoziiert sind. Sie sind beispielsweise in der Lage, hocheffizient das Wachstum oder/und die Ausbreitung der malignen Tumoren sowie die Metastasierung von Tumoren zu hemmen. Dabei können die uPA-Inhibitoren gegebenenfalls zusammen mit anderen Tumormitteln oder mit anderen Behandlungsarten, z.B. Bestrahlung oder chirurgischen Eingriffen, eingesetzt werden. Weiterhin sind die Inhibitoren auch für andere uPA-assoziierte oder/und uPAR assoziierte Erkrankungen wirksam.

Diese Verbindungen sind in der Lage, hocheffizient das Wachstum oder/und die Ausbreitung von malignen Tumoren zu hemmen, z.B. die Tumorausbreitung beim Pankreaskarzinom, das Tumorwachstum des Mammakarzinoms sowie die Metastasierung von Tumoren.

Weiterhin sind die erfindungsgemäßen Inhibitoren auch für andere uPA-assoziierte Erkrankungen wirksam, beispielsweise zur Bekämpfung von Krankheiten wie Arthritis, Entzündungen, Osteoporose, Retinopathien, z.B. altersbedingter Makula Degeneration, bei der Verhinderung der Blasenbildung bei der Hauterkrankung Pemphigus vulgaris.

Die Verabreichung erfolgt vorzugsweise gemeinsam, z.B. als Vor- oder/und Nachbehandlung sowie behandlungsbegleitend im Zusammenhang mit chirurgischen Eingriffen, Strahlenbehandlung oder/und Chemotherapie.

Ein weiterer Gegenstand der Erfindung betrifft die Verwendung eines erfindungsgemäßen Konzentrats zur Herstellung einer zur Injektion oder Infusion geeigneten Wirkstofflösung durch Verdünnen in geeigneten Isotonisierungsmittel, wobei 5-prozentige Glucoselösung bevorzugt verwendet wird und die Wirkstoffkonzentration vorzugsweise bis zu 1 mg/ml beträgt.

Die erfindungsgemäße Formulierung wird zur Bekämpfung von urokinaseassoziierten Erkrankungen, insbesondere zur Tumorbekämpfung, beispielsweise zur Bekämpfung von Mammakarzinom und Pankreaskarzinom oder/und Metastasenbildung, eingesetzt.

Ein weiterer Gegenstand der Erfindung ist ein Verfahren zur Stabilisierung von pharmazeutischen Formulierungen, umfassend eine Verbindung, die eine Amidino-, Hydroxyamidino-, Guanidino- und/oder Hydroxyguanidinogruppe aufweist, vorzugsweise Amidino- und/oder Guanidino-Phenylalanin-Derivate bzw. deren Hydroxyverbindungen, durch Hinzufügen einer geeigneten Menge eines Gemisches aus einem Polyol und einem Alkohol, und eine wässrige Phase, umfassend einen Puffer. Vorzugsweise wird zusätzlich ein Isotonisierungsmittel hinzugefügt.

Der Alkohol, das Polyol, der Puffer und das Isotonisierungsmittel sind wie oben beschrieben.

Als Wirkstoff wird vorzugsweise ein als Urokinase-Inhibitor wirksames Amidino- und/oder Guanidino-phenylalanin-Derivat, wie oben beschrieben, verwendet.

Weiterhin soll die Erfindung im folgenden beispielhaft und keineswegs einschränkend näher erläutert werden.

## Beschreibung der Abbildungen

**Tabelle 1** zeigt die maximale Löslichkeit von WX-UK1 in verschiedenen Solventien und Lösungsmittelgemischen.

**Abbildung 1** zeigt den Verlauf der WX-UK1 Reinheit und des pH-Wertes bei 40 °C in PG/EtOH/Wasser (4/1/5).

**Abbildung 2** zeigt den potenziellen Zerfallsmechanismus von WX-UK1 in wässriger Lösung.

**Abbildung 3** zeigt die pH-Abhängigkeit des WX-UK1-Zerfalls bei 60 °C beobachtet über 48 h.

**Abbildung 4** zeigt die Stabilität der einzelnen WX-UK1 Formulierungen bei 60 °C.

**Abbildung 5** zeigt die Stabilität der gepufferten WX-UK1 Formulierungen im Vergleich zur ungepufferten Lösung.

**Abbildung 6** zeigt die Reinheit und den Gehalt der WX-UK1 Formulierungen bei 40 °C über 5 Monate.

**Beispiele**

**Beispiel 1: pH-Abhängigkeit des Wirkstoffzerfalls**

Um die pH-Abhängigkeit des Wirkstoffzerfalls (WX-UK1) bei drei verschiedenen pH-Werten unter Temperaturbehandlung bei 60 °C zu untersuchen, wurden 2,5 mg WX-UK1 in 1 ml Ethanol/Wasser (1:1 v/v) gelöst. Diese Lösung wurde in drei Gefäße aliquotiert. Ein Aliquot wurde durch Zugabe von 20 µl 1 N Salzsäure auf pH 2, ein zweites Aliquot durch Zugabe von 20 µl 2N Natronlauge auf pH 11 eingestellt und das dritte Aliquot bei neutralem pH belassen. Die Lösungen wurden nach Inkubation für definierte Zeitpunkte (0, 5, 12 und 48 h) mittels einer stabilitätsindizierenden HPLC-Methode analysiert. Es zeigte sich, dass WX-UK1 bei saurem pH für den untersuchten Zeitraum stabil geblieben ist. Bei neutralem bzw. basischem pH zeigte sich jedoch ein moderater bis rascher Zerfall von WX-UK1 (Abbildung 3).

**Beispiel 2: Wirkstoffstabilität in Polyol/Ethanol-Formulierung**

Zu 40 mg WX-UK1 wurden nacheinander 0,4 ml Propylenglykol (PG) und 0,1 ml Ethanol hinzugegeben. Diese Lösung wurde mit 0,3 ml Wasser soweit aufgefüllt und gerührt, bis WX-UK1 in Lösung gegangen ist und die Lösung eine schwache Opaleszenz zeigte. Anschließend wurde das restliche Wasser (ca. 0,2 ml) zugegeben. Anschließend wurde die Lösung bei 40 °C gelagert und der pH-Wert bzw. die Reinheit der Lösung jeweils nach einem Zeitraum von 2, 4, 6, 8 und 12 Wochen analysiert. Hierzu wurde 250 µl der WX-UK1 Lösung in einen 100 ml Messkolben überführt und mit Wasser/Acetonitril (50:50 v/v) bis zur Füllmarke aufgefüllt (Konzentration: ca. 0,1 mg/ml WX-UK1). 20 µl dieser Verdünnung wurde anschließend mittels einer stabilitätsindizierenden HPLC-Methode analysiert (siehe Anhang). Die pH-Wert-Analyse der WX-UK1-Lösung wurde mittels einer potentiometrischen Methode bei 20-25 °C durchgeführt. Es zeigte sich (Abbildung 1), dass WX-UK1 umso schneller zerfällt je höher der pH-Wert der Lösung ist.

**Beispiel 3: Alternative gepufferte WX-UK1 Formulierungen**

Es wurden jeweils 5 ml der folgenden Lösungen (a) bis (e) hergestellt. Anschließend wurden die Lösungen bei 60 °C gelagert und der pH-Wert bzw. die Reinheit der Lösung jeweils nach einem Zeitraum von 0, 12, 24, 48 Stunden analysiert. Hierzu wurden 250 µl der WX-UK1 Lösung in einen 100 ml Messkolben überführt und mit Wasser/Acetonitril (50:50 v/v) bis zur Füllmarke aufgefüllt (Konzentration ist ca. 0,1mg/ml WX-UK1). 20 µl dieser Verdünnung der WX-UK1-Lösung wurde anschließend mittels einer stabilitätsindizierenden HPLC-Methode analysiert (siehe Anhang).

a) 1 mg/ml WX-UK1 in Wasser (pH-Wert messen)
b) 40 mg/ml WX-UK1 in PG/Ethanol/Wasser; 4:1:5 (pH-Wert messen)
c) 40 mg/ml WX-UK1 in 1,2-Propandiol/Ethanol, wasserfrei
d) 40 mg/ml WX-UK1 in PG/Ethanol/ 40 mM Natriumcitrat; 4:1:5 (pH-Wert auf den der Lösung (b) einstellen)
e) 40 mg/ml WX-UK1 in PG/Ethanol/ 40 mM Natrium-Acetatpuffer; 4:1:5 (pH-Wert auf den der Lösung (b) einstellen)

Zur Herstellung von 80 mM (pH-Wert wie oben) Natriumcitratpuffer wurden 1,68 g Citronensäure-monohydrat in 8 ml 1N Natriumhydroxid aufgenommen und mit Wasser auf 100 ml aufgefüllt. Der pH-Wert wurde mit 80 nM Natriumhydroxid eingestellt. 1 mM Puffer ergab sich durch eine 1/80 Verdünnung und nachfolgender pH-Einstellung.

Natriumacetat und Natriumcitrat wurden bei pH 5 als parenteral verträgliches System zur Pufferung der WX-UK1 Formulierung ausgewählt und die chemische und physikalische Stabilität gegen die nicht gepufferte Formulierung und eine wasserfreie Formulierung getestet. Von einer Phosphatpufferung wurde abgesehen, da aus früheren Untersuchungen bekannt war, dass sich in WX-UK1-Lösungen durch Zugabe von Phosphatpuffer ein Niederschlag bildet. Die Stabilitätsstudie wurde bei 60 °C durchgeführt, um einen rascheren Zerfall von WX-UK1 zu erreichen. In der Citrat-gepufferten WX-UK1-Lösung zeigte sich sehr rasch ein Niederschlag, der zusätzlich zum Überstand der Lösung auf die Reinheit von WX-UK1 untersucht wurde. Wegen der mangelnden physikalischen Stabilität der Natriumcitrat gepufferten Lösung und der mangelnden chemischen Stabilität der untersuchten Formulierung wurde die Stabilitätsstudie für diese Formulierungen nach vier Tagen abgebrochen. Die größte physikalische und chemische Stabilität zeigte die Natriumacetat gepufferte WX-UK1 Formulierung. Ausgewertet wurden die WX-UK1 Peakflächenprozent, ermittelt durch eine stabilitätsindizierende HPLC-Methode (Abbildung 4).

**Beispiel 4: Stabilität von Natriumacetat gepufferten Lösungen**

Natriumacetat in verschiedenen Molaritäten bei pH 5 wurde aufgrund dieser Ergebnisse als parenteral verträgliches System zur Pufferung der WX-UK1 Formulierung ausgewählt und zusätzlich die chemische Stabilität gegen die nicht gepufferte Formulierung getestet. Die Stabilitätsstudie wurde bei 60 °C durchgeführt, um einen beschleunigten Zerfall von WX-UK1 zu erreichen (Abbildung 5). Ausgewertet wurden die WX-UK1 Peakflächenprozent, ermittelt durch eine stabilitätsindizierende HPLC-Methode.

Ergebnis: Durch eine Pufferung der Formulierung kann der Zerfall von WX-UK1 erheblich verlangsamt werden.

**Beispiel 5: Stabilitätsstudie über mehrere Monate mit einer Natriumacetat gepufferten Formulierung**

Herstellung eines 200 mM Natriumacetat-Puffers:

821 mg Natriumacetat wurden in 50 ml Wasser gelöst und mit konzentrierter Essigsäure auf pH 5 eingestellt und die fertige Pufferlösung durch einen Spritzenfilter Millipore, Millex GV 0,22 μm filtriert.

Herstellung der 100 mM Natriumacetat-gepufferten WX-UK1 Formulierung:

960 mg WX-UK1 wurden in ein Gefäß eingewogen und 9,6 ml Propylenglykol hinzugegeben, anschließend 2,4 ml Ethanol. Mit 7 ml 200 mM Natriumacetat-Puffer wurde diese Lösung soweit aufgefüllt und gerührt bis WX-UK1 in Lösung gegangen ist und die Lösung eine schwache Opaleszenz zeigte. Anschließend wurde der restliche Natriumacetat-Puffer (5 ml) zugegeben. Die Lösungen wurden zu je 1 ml aliquotiert und anschleißend bei 40 °C gelagert: Die Reinheit und der WX-UK1 Gehalt der Lösung wurden jeweils nach einem Zeitraum von 2, 4, 6, 8 und 12 Wochen analysiert. Hierzu wurden 250 μl der WX-UK1 Lösung in einen 100 ml Messkolben überführt und mit Wasser/Acetonitril (50:50 v/v) bis zur Füllmarke aufgefüllt (Konzentration: ca. 0,1 mg/ml WX-UK1). 20 μl dieser Verdünnung der WX-UK1 Lösung wurden anschließend mittels einer stabilitätsindizierenden HPLC-Methode analysiert (siehe Anhang).

Der WX-UK1 Gehalt wird nach folgender Formel gegen zwei WX-UK1 Standard-Lösungen ausgewertet:

$$\text{WX-UK1 [mg/ml]} = \frac{\text{Area}_{PL} \times (W_{St1} + W_{St2}) \times C_{St}}{(\text{Area}_{St1} + \text{Area}_{St2}) \times 100 \times V_{PL}}$$

| | | | |
|---|---|---|---|
| $\text{Area}_{PL}$ | = | Fläche Prüflösung (WX-UK1 Peak) | [mAU*s] |
| $\text{Area}_{St1}$ | = | Fläche Standard I (WX-UK1 Peak) | [mAU*s] |
| $\text{Area}_{St2}$ | = | Fläche Standard II (WX-UK1 Peak) | [mAU*s] |
| $W_{St1}$ | = | Einwaage Standard I | [mg] |
| $W_{St2}$ | = | Einwaage Standard II | [mg] |
| $C_{St}$ | = | Gehalt des Standards | [%] |
| $V_{PL}$ | = | Volumen der eingesetzten Injektionslösung | [ml] |

In Abbildung 6 ist sowohl die Reinheit der Formulierungen als auch der Gehalt an WX-UK1 im zeitlichen Verlauf dargestellt. Es wir deutlich, dass sowohl die Reinheit als auch der Gehalt der gepufferte WX-UK1 Formulierung im Gegensatz zur ungepufferten sehr stabil bleibt (Abbildung 6).

**Beispiel 6: Die WX-UK1 Formulierung (40 mg/ml) wird nach folgendem Schema hergestellt:**

| | |
|---|---|
| WX-UK1 | 40 mg |
| Propylenglykol | 0.4 ml |
| Ethanol, absolut | 0.1 ml |
| Wasser | auf 1 ml auffüllen |

WX-UK1 wurde in ein Gefäß eingewogen und Propylenglykol hinzugegeben, anschließend Ethanol. Mit 0,3 ml Wasser wurde diese Lösung aufgefüllt und gerührt bis WX-UK1 in Lösung gegangen war bzw. die Lösung eine schwache Opaleszenz zeigte. Anschließend wurde das restliche Wasser zugegeben.

**Beispiel 7: Die Natriumacetat gepufferte WX-UK1 Formulierung (40mg/ ml) wurde nach folgendem Schema hergestellt:**

| | |
|---|---|
| WX-UK1 | 40 mg |
| Propylenglykol | 0.4 ml |
| Ethanol, absolut | 0.1 ml |
| Natriumacetatpuffer (200mM) | auf 1 ml auffüllen |

WX-UK1 wurde in ein Gefäß eingewogen und Propylenglykol hinzugegeben, anschließend Ethanol. Diese Lösung wurde mit 0,3 ml 200 mM Natriumacetat aufgefüllt und solange gerührt bis WX-UK1 vollständig in Lösung gegangen ist bzw. die Lösung eine schwache Opaleszenz zeigte. Anschließend wurde der restliche Acetatpuffer zugegeben.

**Beispiel 8: Stabilitätsindizierende HPLC-Methode zur Überprüfung der Stabilität der WX-UK1 Formulierungen:**

| | | |
|---|---|---|
| Apparaturen und Bedingungen: | HPLC – Anlage: | Agilent 1100 |
| | Säulentyp: | LUNA C8(2) 5 µm, 250 mm, 4,6 mm ID oder Kromasil 100 C18 5 µm, 250 mm, 4 mm ID |
| | Detektionswellenlänge: | UV 205 nm |
| | Probengebertemperatur: | 4° C |
| | Säulentemperatur: | 40° C |
| | Einspritzvolumen: | 20 µl |
| | Art des Systems: | Gradientensystem |
| | Flussrate: | 1 ml/min |
| | Laufzeit: | 44 min |
| | Laufmittel: | A: Puffer pH = 5,00 ± 0,05 (25 mM Na-Phosphat)<br>B: Acetonitril |

Gradient:

| Zeit [min] | A [%] | B [%] | |
|---|---|---|---|
| 0 | 75 | 25 | |
| 30 | 28 | 72 | |
| 31 | 10 | 90 | |
| 36 | 10 | 90 | |
| 37 | 75 | 25 | |
| 44 | 75 | 25 | end |

| | |
|---|---|
| Einzusetzende Materialien: | Wasser z. C. (zur Chromatographie; gradient grade)<br>Acetonitril z. C.<br>Ortho-Phosphorsäure 85 % Suprapur<br>di-Natriumhydrogenphosphat z. A. (>99.99 %)<br>25 mM Natriumphosphatpuffer pH = 5,00 ± 0,05<br>(3.55 g $Na_2HPO_4$ wurden mit Wasser auf 1000 ml aufgefüllt. Der pH-Wert wurde mit $H_3PO_4$ auf pH = 5,00 ± 0,05 eingestellt) |

Probenaufbereitung:

WX-UK1 Prüflösung: 25 µl WX-UK1 (40mg/ml) Konzentrat wurden mit 975 µl Wasser/Acetonitril (50:50 v/v) verdünnt. 100 µl dieser Verdünnungslösung wurden mit 900 µl Wasser/Acetonitril (50:50 v/v) verdünnt (c ist ca. 0,1 mg/ml WX-UK1).

**Beispiel 9: HPLC-MS Methode zur Identifizierung der Zerfallsprodukte der WX-UK1 Formulierungen:**

| | | |
|---|---|---|
| Apparaturen und Bedingungen: | HPLC – Anlage: | Waters Alliance: 2695 Separation Module; 2487 UV-VIS Detektor |
| | MS-Detektor: | Micromass ZQ: Single Quadrupol MS-Detektor |
| | Säulentyp: | Symmetry C18 3,5 µm; 2,1 x 100 mm |
| | Detektionswellenlänge: | UV 215 nm |
| | Probengebertemperatur: | 4 °C |

| | | |
|---|---|---|
| Säulentemperatur: | 35 °C | |
| Einspritzvolumen: | 20 µl | |
| Art des Systems: | Gradientensystem | |
| Flussrate: | 0,5 ml/min | |
| Laufzeit: | 16 min | |
| Laufmittel: | A: $NH_4Ac$-Puffer/ACN 72/25 (v/v)<br>B: $NH_4Ac$-Puffer/ACN 30/70 (v/v)<br>C: Methanol | |
| Gradient: | (siehe Tabelle) | |

| Zeit [min] | A [%] | B [%] | C [%] | |
|---|---|---|---|---|
| 0 | 100 | 0 | 0 | |
| 10 | 0 | 100 | 0 | |
| 11 | 0 | 20 | 80 | |
| 12 | 0 | 20 | 20 | |
| 13 | 100 | 0 | 0 | |
| 16 | 100 | 0 | 0 | end |

| | |
|---|---|
| Einzusetzende Materialien: | Wasser z. C. (zur Chromatographie; gradient grade)<br>Acetonitril z. C. (zur Chromatographie; gradient grade)<br>Methanol (HPLC gradient grade)<br>Eisessig (Suprapur)<br>Ammoniumacetat (HPLC grade)<br>50 mM $NH_4Ac$: 3,85 g $NH_4Ac$ auf 1000 ml mit Wasser auffüllen. pH Einstellung auf 5 mit Eisessig |

**Patentansprüche**

1. Pharmazeutische Formulierung umfassend

(i) ein Amidino-, Hydroxyamidino-, Guanidino- und/oder Hydroxyguanidinophenylalanin-Derivat als Wirkstoff,
(ii) ein Gemisch aus Polyol/Alkohol und
(iii) eine wässrige Phase, umfassend einen Puffer.

2. Pharmazeutische Formulierung nach Anspruch 1, wobei der Puffer ein Acetatpuffer ist, insbesondere ein Natriumacetatpuffer ist.

3. Pharmazeutische Formulierung nach einem der vorhergehenden Ansprüche, wobei der Alkohol Ethanol ist.

4. Pharmazeutische Formulierung nach einem der vorhergehenden Ansprüche, wobei das Polyol ausgewählt ist aus Glycerin, Propylenglycol und Polyethylenglycol.

5. Pharmazeutische Formulierung nach einem der vorhergehenden Ansprüche, wobei der Alkohol und/oder das Polyol zu etwa 20-60 % bezogen auf die gesamte Formulierung vorhanden ist.

6. Pharmazeutische Formulierung nach einem der vorhergehenden Ansprüche, wobei die Komponente (ii) ein Gemisch aus Polyol/Alkohol in einem Mischungsverhältnis von 2:1 bis 10:1 umfasst.

7. Pharmazeutische Formulierung nach einem der vorhergehenden Ansprüche, weiterhin umfassend ein Isotonisierungsmittel und/oder weitere Hilfsstoffe oder Kombinationen davon.

8. Pharmazeutische Formulierung nach einem der vorhergehenden Ansprüche, wobei der Wirkstoff ausgewählt ist aus Nα-(2,4,6-Triisopropylphenylsulfonyl)-3-amidino-(D,L)-phenylalanin-4-ethoxy-carbonylpiperazid, Nα-(2,4,6-Triisopropyl -phenylsulfonyl)-3-guanidino-(D,L)-phenylalanin-4-ethoxycarbonylpiperazid, den L-Enantiomeren davon oder Nα-(2,4,6-Triisopropylphenyl-sulfonyl)-3-amidino-(L)-phenylalanin-4-ethoxycarbonylpiperazid, das Chlorid, Hydrogensulfat oder/und Sulfatsalz davon und den pharmazeutisch verträglichen Salzen davon.

9. Konzentrat einer pharmazeutischen Formulierung nach einem der vorhergehenden Ansprüche, wobei der Wirkstoff in einer Konzentration von bis zu 100 mg/ml und der Puffer in einer Konzentration von bis zu 1000 mM vorliegt.

10. Formulierung oder Konzentrat nach einem der vorhergehenden Ansprüche zur Verwendung in der Bekämpfung von Urokinase-assoziierten Erkrankungen, vorzugsweise zur Tumorbekämpfung, Tumorprävention, Bekämpfung oder/und Prävention der Metastasenbildung, besonders bevorzugt zur Bekämpfung und/oder Prävention von Mammakarzinom, Pankreaskarzinom oder/und der Metastasenbildung.

11. Formulierung oder Konzentrat nach einem der vorhergehenden Ansprüche 1 bis 9 zur Bekämpfung und/oder Prävention von Arthritis, Entzündungen, Osteoporose, Retinopathien, z.B. altersbedingter Makula Degeneration, bei der Verhinderung der Blasenbildung bei der Hauterkrankung Pemphigus vulgaris.

12. Verwendung einer pharmazeutischen Formulierung oder eines Konzentrats nach einem der vorhergehenden Ansprüche zur Herstellung einer zur Verabreichung durch Injektion oder Infusion geeigneten Lösung durch Verdünnen der pharmazeutischen Formulierung oder des Konzentrats in einem geeigneten Isotonisierungsmittel, wobei die maximale Konzentration des Wirkstoffs bis zu 1 mg/ml beträgt.

13. Verwendung einer pharmazeutischen Formulierung oder eines Konzentrats nach einem der Ansprüche 1 bis 9 zur Herstellung eines Medikaments zur Behandlung und/oder Prävention einer Tumorerkrankung und/oder zur Behandlung und/oder Prävention von Metastasenbildung.

14. Kombination enthaltend eine pharmazeutische Formulierung oder ein Konzentrat nach einem der Ansprüche 1 bis 9 und ein zytostatisches oder/und zytotoxisches Mittel.

15. Verfahren zur Stabilisierung von pharmazeutischen Formulierungen, umfassend als Wirkstoff eine Verbindung, die eine Amidino-, Hydroxyamidino, Guanidino- und/oder Hydroxyguanidinogruppe aufweist, durch Hinzufügen einer geeigneten Menge eines Gemisches aus Polyol/Alkohol und einer geeigneten Menge einer wässrigen Phase, umfassend einen Puffer zu dem Wirkstoff.

**Tabelle 1**

| **Solvens** | **Löslichkeit WX-UK1 [mg/ml]** |
|---|---|
| Wasser | 5,2 |
| 5% D-Glucose | 6,6 |
| 0,9% NaCl-Lösung | 1,3 |
| Propylenglykol | > 100 |
| Polyethylenglykol 400 | > 100 |
| 10% Propylenglykol in Wasser | 16 |
| 40% Propylenglykol in Wasser | 78 |

(fortgesetzt)

| Solvens | Löslichkeit WX-UK1 [mg/ml] |
|---|---|
| 10% Ethanol in Wasser | 22 |
| Propylenglykol/Ethanol/Ethanol/Wasser 60/15/25 | > 108 |
| Propylenglykol/Ethanol/Wasser 40/10/50 | > 100 |
| Propylenglykol/Ethanol/Wasser 20/5/75 | 47 |
| Propylenglykol/Ethano/Ethanol/Wasser 10/10/80 | 38 |

## Claims

**1.** Pharmaceutical formulation comprising

(i) an amidino, hydroxyamidino, guanidino and/or hydroxyguanidinophenylalanine derivative as an active ingredient,
(ii) a mixture of a polyol and an alcohol and
(iii) an aqueous phase comprising a buffer.

**2.** Pharmaceutical formulation according to claim 1, wherein the buffer is an acetate buffer and in particular a sodium acetate buffer.

**3.** Pharmaceutical formulation according to one of the previous claims, wherein the alcohol is ethanol.

**4.** Pharmaceutical formulation according to one of the previous claims, wherein the polyol is selected from glycerol, propylene glycol and polyethylene glycol.

**5.** Pharmaceutical formulation according to one of the previous claims, wherein the alcohol and/or the polyol is present in an amount of about 20 - 60 % based on the entire formulation.

**6.** Pharmaceutical formulation according to one of the previous claims, wherein the component (ii) comprises a mixture of polyol/alcohol in a mixing ratio of 2:1 to 10.1.

**7.** Pharmaceutical formulation according to one of the previous claims, additionally comprising an isotonizing agent and/or additional auxiliary substances or combinations thereof.

**8.** Pharmaceutical formulation according to one of the previous claims, wherein the active ingredient is selected from Nα-(2,4,6-triisopropylphenylsulfonyl)-3-amidino-(D,L)-phenylalanine-4-ethoxy-carbonylpiperazide, Nα-(2,4,6-triisopropyl-phenylsulfonyl)-3-guanidino-(D,L)-phenylalanine-4-ethoxycarbonylpiperazide, the L-enantiomers thereof or Nα-(2,4,6-triisopropylphenylsulfonyl)-3-amidino-(L)-phenylalanine-4-ethoxycarbonyl piperazide, the chloride, hydrogen sulfate or/and sulfate salt thereof and pharmaceutically acceptable salts thereof.

**9.** A concentrate of a pharmaceutical formulation according to one of the previous claims, wherein the active ingredient is present at a concentration of up to 100 mg/ml and the buffer is present at a concentration of up to 1000 mM.

**10.** A formulation or concentrate according to one of the previous claims for use in combating urokinase-associated diseases, preferably for combating tumours, for tumour prevention, for combating or/and preventing the formation of metastases, particularly preferably for combating and/or preventing mammary carcinoma, pancreatic carcinoma or/and the formation of metastases.

**11.** A formulation or concentrate according to one of the previous claims 1 to 9 for combating and/or preventing arthritis, inflammation, osteoporosis, retinopathies, for example age-related macular degeneration, for preventing blistering in the skin disease pemphigus vulgaris.

**12.** Use of a pharmaceutical formulation or a concentrate according to one of the previous claims for producing a solution suitable for administration by injection or infusion by diluting the pharmaceutical formulation or the concentrate in a

suitable isotonising agent, wherein the maximum concentration of the active ingredient is up to 1 mg/ml.

**13.** Use of a pharmaceutical formulation or a concentrate according to one of the claims 1 to 9 for the production of a medicament for treating and/or preventing a tumour disease and/or for treating and/or preventing the formation of metastases.

**14.** Combination containing a pharmaceutical formulation or a concentrate according to one of the claims 1 to 9 and a cytostatic or/and cytotoxic agent.

**15.** Method for stabilizing pharmaceutical formulations containing a compound which has an amidino, hydroxyamidino, guanidino and/or hydroxyguanidino group as an active ingredient by adding a suitable amount of a mixture of polyol/alcohol and a suitable amount of an aqueous phase comprising a buffer to the active ingredient.

## Revendications

**1.** Formulation pharmaceutique comprenant

(i) un dérivé d'amidino-, hydroxyamidino-, guanidino- et/ou hydroxyguanidino-phénylalanine comme principe actif,
(ii) un mélange de polyol/alcool et
(iii) une phase aqueuse comprenant un tampon.

**2.** Formulation pharmaceutique selon la revendication 1 où le tampon est un tampon acétate, en particulier un tampon acétate de sodium.

**3.** Formulation pharmaceutique selon l'une des revendications précédentes où l'alcool est l'éthanol.

**4.** Formulation pharmaceutique selon l'une des revendications précédentes où le polyol est choisi parmi la glycérine, le propylèneglycol et le polyéthylèneglycol.

**5.** Formulation pharmaceutique selon l'une des revendications précédentes où l'alcool et/ou le polyol est présent à raison d'environ 20 - 60 % par rapport à la formulation totale.

**6.** Formulation pharmaceutique selon l'une des revendications précédentes où le composant (ii) comprend un mélange de polyol/alcool dans un rapport de mélange de 2 : 1 à 10 : 1.

**7.** Formulation pharmaceutique selon l'une des revendications précédentes comprenant en outre un agent d'isotonie et/ou d'autres adjuvants ou des combinaisons de ceux-ci.

**8.** Formulation pharmaceutique selon l'une des revendications précédentes où le principe actif est choisi parmi le N$\alpha$-(2,4,6-triisopropylphénylsulfonyl)-3-amidino-(D,L)-phénylalanine-4-éthoxycarbonyl-pipérazide, le N$\alpha$-(2,4,6-triisopropylphénylsulfonyl)-3-guanidino-(D,L)-phényl-alanine-4-éthoxycarbonylpipérazide, leurs énantiomères L ou le N$\alpha$-(2,4,6-triisopropylphénylsulfonyl)-3-amidino-(L)-phénylalanine-4-éthoxycarbonyl-pipérazide, son chlorure, hydrogénosulfate et/ou sel sulfate et ses sels pharmaceutiquement acceptables.

**9.** Concentré d'une formulation pharmaceutique selon l'une des revendications précédentes où le principe actif est présent en une concentration pouvant atteindre 100 mg/ml et le tampon en une concentration pouvant atteindre 1000 mM.

**10.** Formulation ou concentré selon l'une des revendications précédentes pour une utilisation dans la lutte contre les maladies associées à l'urokinase, de préférence pour la lutte contre les tumeurs, la prévention des tumeurs, la lutte contre et/ou la prévention de la formation de métastases, de manière particulièrement préférée pour la lutte contre et/ou la prévention du cancer du sein, du cancer du pancréas et/ou de la formation de métastases.

**11.** Formulation ou concentré selon l'une des revendications 1 à 9 précédentes pour la lutte contre et/ou la prévention de l'arthrite, des inflammations, de l'ostéoporose, des rétinopathies, par exemple de la dégénérescence maculaire liée à l'âge, pour éviter la formation de vésicules dans la maladie de peau pemphigus vulgaris.

**12.** Utilisation d'une formulation pharmaceutique ou d'un concentré selon l'une des revendications précédentes pour la production d'une solution appropriée à l'administration par injection ou perfusion par dilution de la formulation pharmaceutique ou du concentré dans un agent d'isotonie approprié, où la concentration maximale du principe actif peut atteindre 1 mg/ml.

**13.** Utilisation d'une formulation pharmaceutique ou d'un concentré selon l'une des revendications 1 à 9 pour la production d'un médicament pour le traitement et/ou la prévention d'une maladie tumorale et/ou pour le traitement et/ou la prévention de la formation de métastases.

**14.** Combinaison contenant une formulation pharmaceutique ou un concentré selon l'une des revendications 1 à 9 et un agent cytostatique et/ou cytotoxique.

**15.** Procédé pour la stabilisation de formulations pharmaceutiques comprenant comme principe actif un composé qui comporte un groupe amidino, hydroxyamidino, guanidino et/ou hydroxyguanidino, par addition d'une quantité appropriée d'un mélange de polyol/alcool et d'une quantité appropriée d'une phase aqueuse comprenant un tampon au principe actif.

**Abbildung 1**

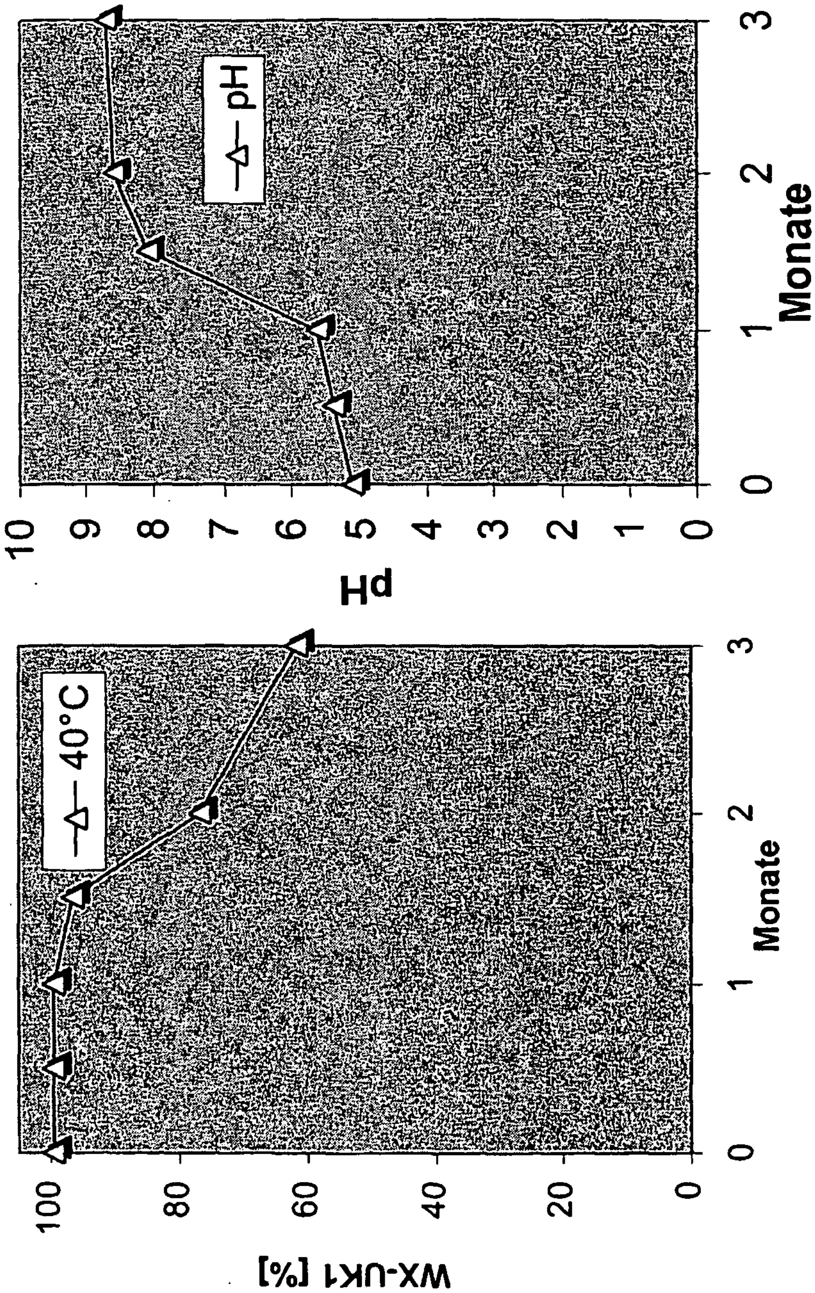

## Abbildung 2

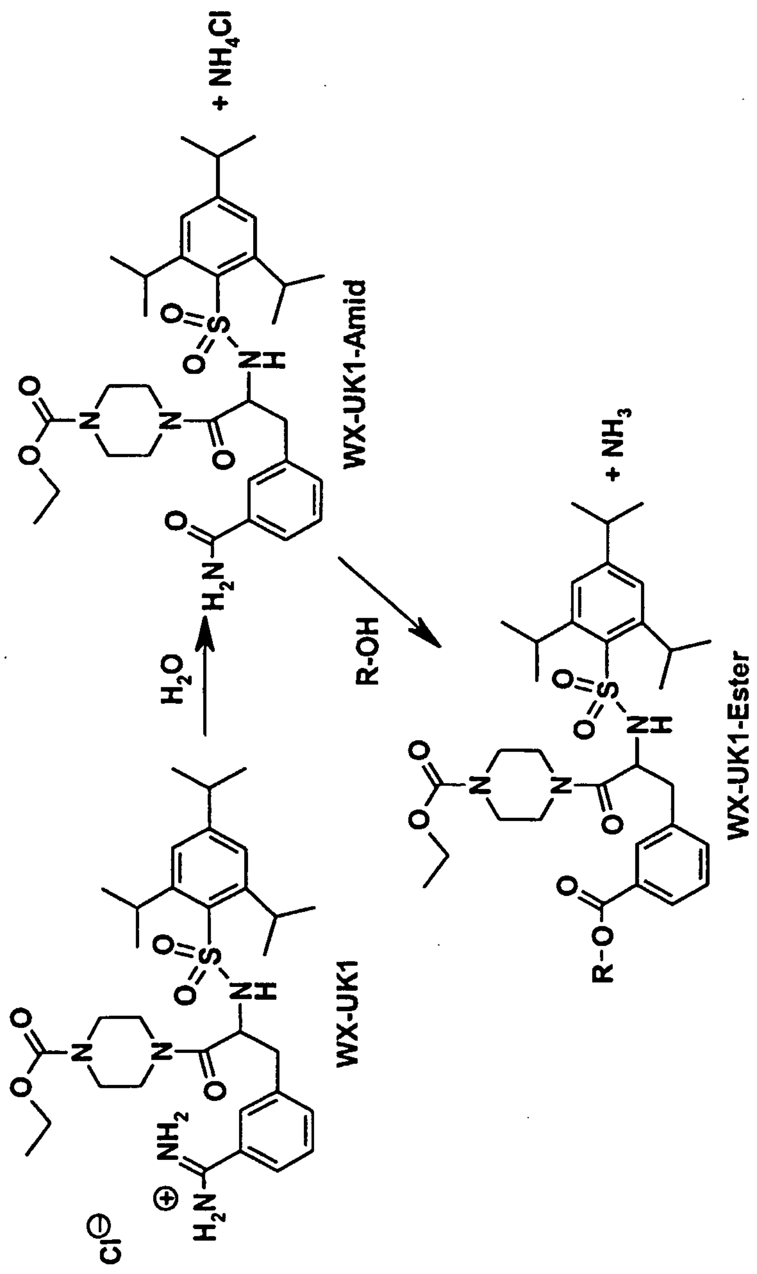

**Abbildung 3**

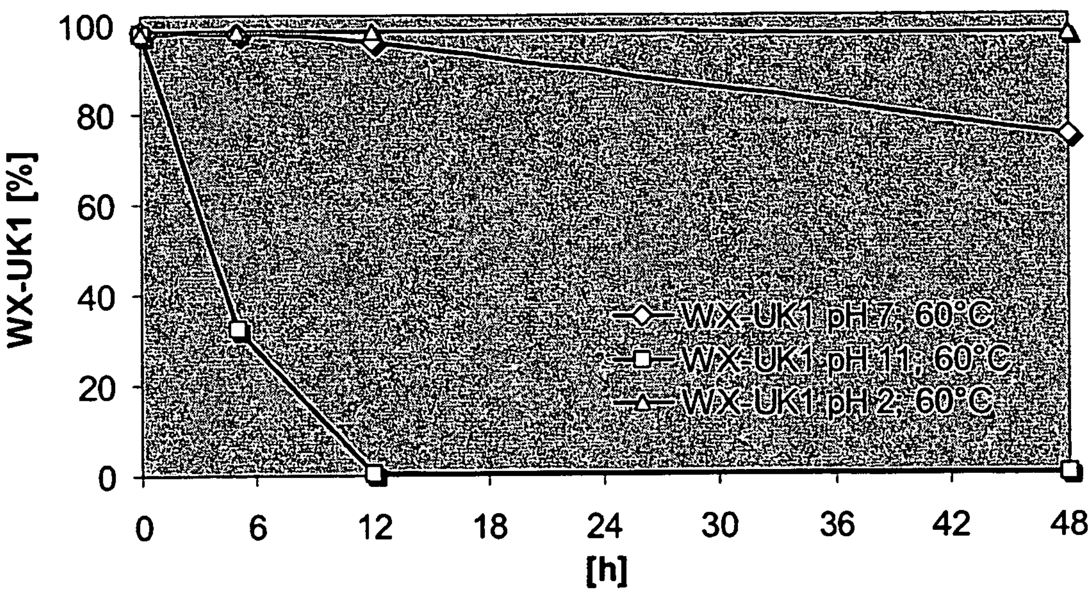
100
80
60
40
20
0
WX-UK1 [%]
0
6
12
18
24
30
36
42
48
[h]
WX-UK1 pH 7; 60°C
WX-UK1 pH 11; 60°C
WX-UK1 pH 2; 60°C

**Abbildung 4**

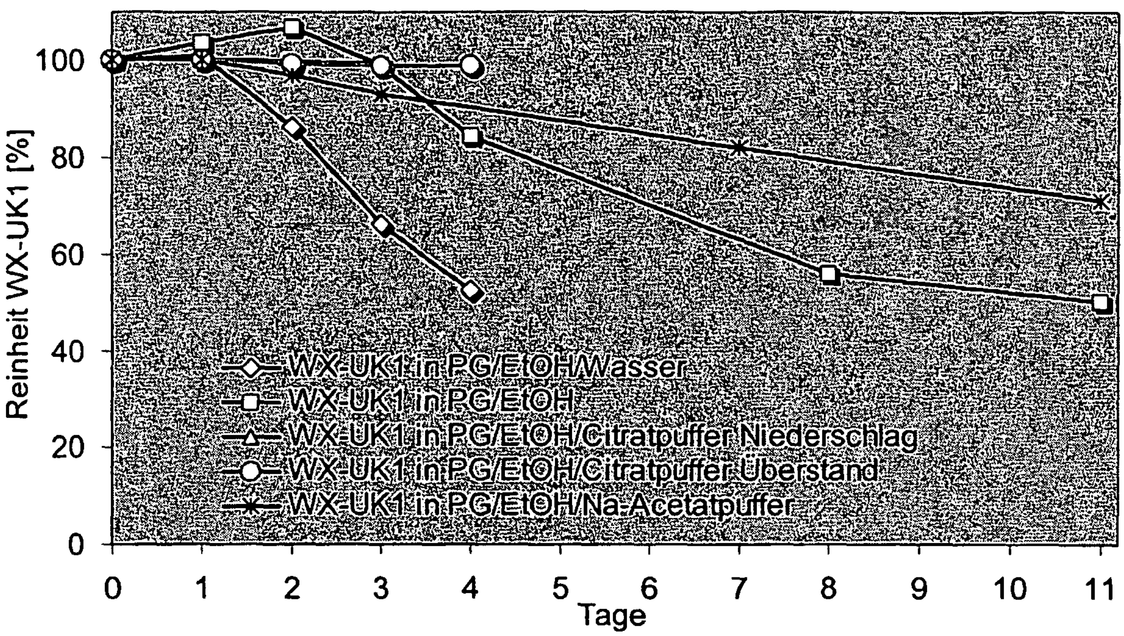
100
80
60
40
20
0
Reinheit WX-UK1 [%]
WX-UK1 in PG/EtOH/Wasser
WX-UK1 in PG/EtOH
WX-UK1 in PG/EtOH/Citratpuffer Niederschlag
WX-UK1 in PG/EtOH/Citratpuffer Überstand
WX-UK1 in PG/EtOH/Na-Acetatpuffer
0
1
2
3
4
5
6
7
8
9
10
11
Tage

**Abbildung 5**

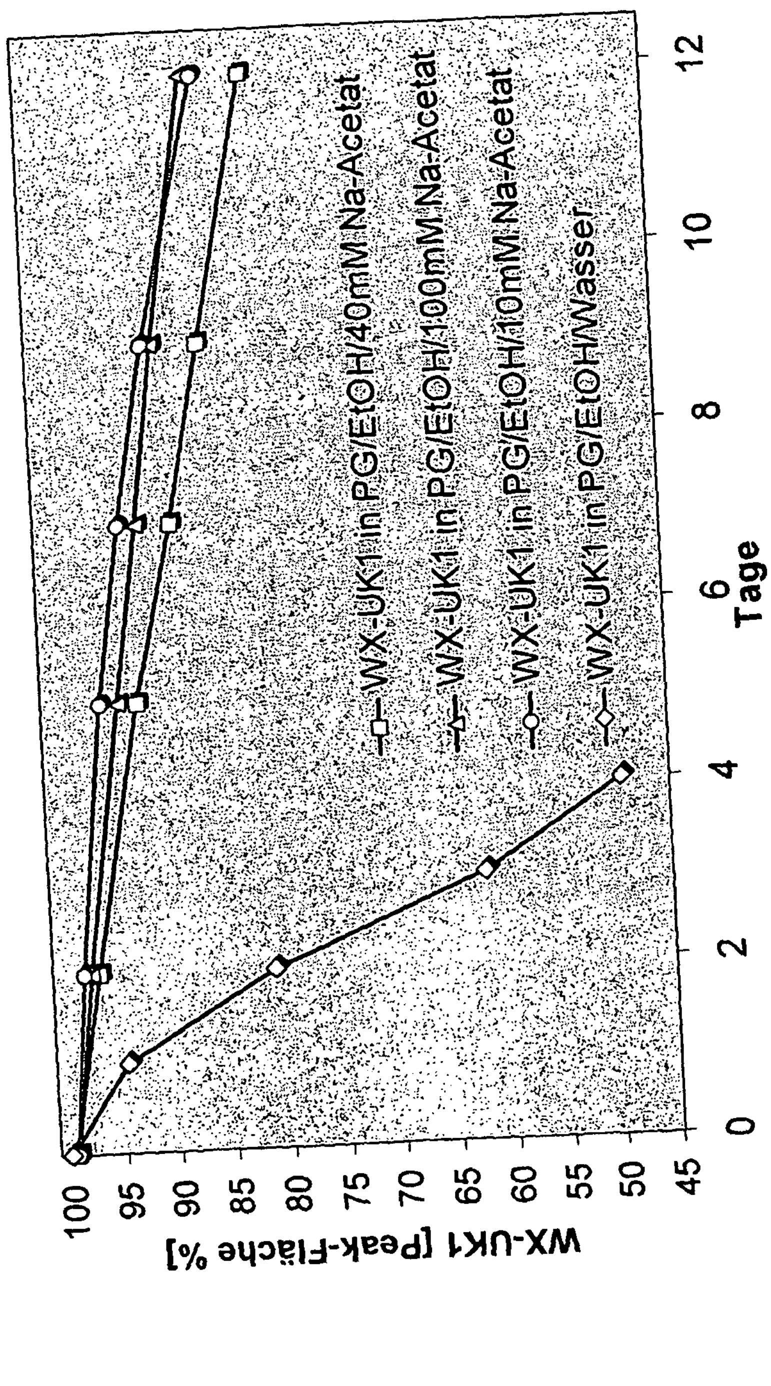
WX-UK1 [Peak-Fläche %]
100
95
90
85
80
75
70
65
60
55
50
45
0
2
4
6
8
10
12
Tage
WX-UK1 in PG/EtOH/40mM Na-Acetat
WX-UK1 in PG/EtOH/100mM Na-Acetat
WX-UK1 in PG/EtOH/10mM Na-Acetat
WX-UK1 in PG/EtOH/Wasser

**Abbildung 6**

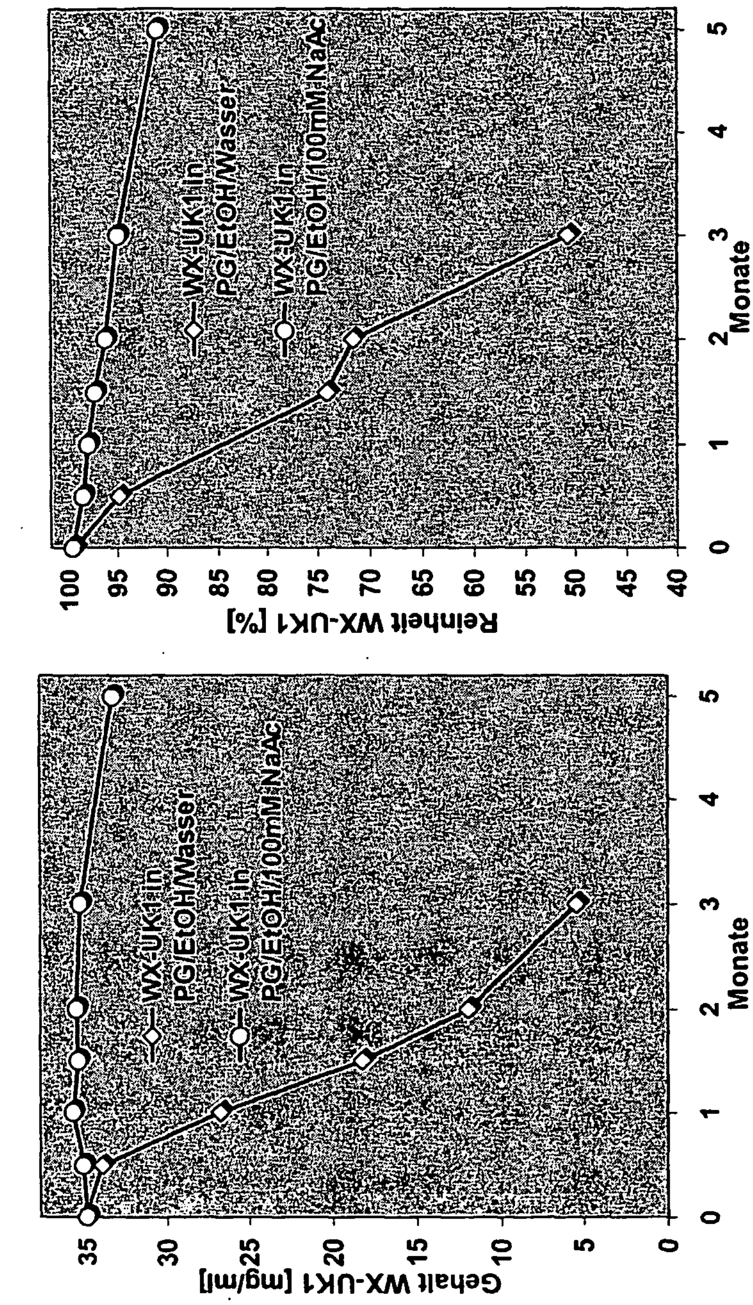
Reinheit WX-UK1 [%]
Monate
WX-UK1 in PG/EtOH/Wasser
WX-UK1 in PG/EtOH/100mM NaAc
Gehalt WX-UK1 [mg/ml]
Monate
WX-UK1 in PG/EtOH/Wasser
WX-UK1 in PG/EtOH/100mM NaAc

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1098651 A
- WO 02074756 A
- WO 03103644 A
- WO 2004011004 A
- EP 2004005682 W


### In der Beschreibung aufgeführte Nicht-Patentliteratur


- The Urokinase/Urokinase receptor system: A new target for cancer therapy?. **Wilhelm et al.** Prospects in Diagnosis and Treatment of Cancer. International Congress Series, Excerpta Medica 1050. Elsevier, 1994, 145-156
- **Hollas et al.** *Cancer Res.,* 1991, vol. 51, 3690-3695
- **Cajot et al.** *Proc. Natl. Acad. Sci. USA,* 1990, vol. 87, 6939-6943
- **Baker et al.** *Cancer Res.,* 1990, vol. 50, 4676-4684
- **Cohen et al.** *Blood,* 1991, vol. 78, 479-487
- **Kobayashi et al.** *Br. J. Cancer,* 1993, vol. 67, 537-544
- **Kook.** *EMBO J.,* 1994, vol. 13, 3983-3991
- **Liu et al.** *Int. J. Cancer,* 1995, vol. 60, 501-506
- **Ossowski ; Reich.** *Cell,* 1983, vol. 35, 611-619
- **Crowley et al.** *Proc. Natl. Acad. Sci. USA,* 1993, vol. 90, 5021-5025
- **Wilhelm et al.** *Clin. Exp. Metast.,* 1995, vol. 13, 296-302
- **Schmitt et al.** *J. Obstet. Gynaecol.,* 1995, vol. 21, 151-165
- **Jaenicke et al.** *Breast Cancer Res. Treat.,* 1993, vol. 24, 195-208
- **Kuhn et al.** *Gynecol. Oncol.,* 1994, vol. 55, 401-409
- **Nekarda et al.** *Lancet,* 1994, vol. 343, 117
- **Pedersen et al.** *Cancer Res.,* 1994, vol. 54, 4671-4675
- **Duggan et al.** *Int. J. Cancer,* 1995, vol. 61, 597-600
- **Ronne et al.** *Breast Cancer Res. Treat.,* 1995, vol. 33, 199-207
- **Heiss et al.** *J. Clin.Oncol.,* 1995, vol. 13, 2084-2093
- **Heiss et al.** *Nature Medicine,* 1995, vol. 1, 1035-1039